# EUROPEAN PATENT SPECIFICATION

(11) **EP 3 107 599 B1**
(45) Date of publication and mention of the grant of the patent: **01.09.2021**
(21) Application number: 15752771.4
(22) Date of filing: 18.02.2015
(51) Int. Cl.: B01J 20/08, B01J 20/06, B01J 20/20, B01J 20/28, B01J 20/02, A61M 1/16, A61M 1/14

(54) **MODULE FOR IN-LINE RECHARGING OF SORBENT MATERIALS WITH OPTIONAL BYPASS**
MODUL ZUM INLINE-WIEDERAUFFÜLLEN EINES SORPTIONSMATERIALS MIT WAHLWEISEM BYPASS
MODULE DE RECHARGE EN LIGNE DE SUBSTANCES SORBANTES À DÉRIVATION FACULTATIVE

(30) Priority: 19.02.2014 US 201461941672 P; 26.02.2014 US 201461945064 P; 30.04.2014 US 201414259665
(43) Date of publication of application: 28.12.2016
(73) Proprietor: Medtronic, Inc., Minneapolis, Minnesota 55432 (US)
(72) Inventor: PUDIL, Bryant J., Plymouth, MN 55447 (US); GERBER, Martin T., Maple Grove, MN 55369 (US); LURA, David B., Maple Grove, MN 55311 (US); MEYER, Thomas E., Stillwater, MN 55082 (US)
(74) Representative: Dehns
(86) International application number: PCT/US2015/016273
(87) International publication number: WO 2015/126879

(56) References cited:
- WO-A1-2013/025957
- WO-A2-2013/028809
- US-A1- 2005 274 658
- US-A1- 2009 101 552
- US-A1- 2011 048 949
- US-A1- 2011 297 593
- US-A1- 2013 213 890
- None

## Description

### FIELD OF THE INVENTION

The invention relates to modules for in-line recharging of sorbent materials in a sorbent cartridge. The modules can be detachable, and the sorbent materials can include zirconium phosphate.

### BACKGROUND

Dialysis involves the movement of blood through a dialyzer that has a semi-permeable membrane. Simultaneously, dialysate is circulated through the dialyzer on an opposite side of the semi-permeable membrane. Toxins present in the blood stream of the patient pass from the blood through the membrane into the dialysate. After passing through the dialyzer, the spent dialysate is discarded. Disposal of spent dialysate requires a large amount of source water for preparing the replacement dialysate necessary for use during continuous dialysis. However, in sorbent dialysis systems, the spent dialysate is re-circulated through a sorbent cartridge rather than being discarded. The sorbent cartridge contains layers of sorbent material which selectively remove specific toxins, or break down toxins, in the dialysate.

The advantage of sorbent dialysis is that a much lower amount of water is required. In four hours of traditional dialysis, up to 120 L of water may be required to generate the dialysate. By contrast, using sorbent dialysis, as little as 6 or 7 L of water may be necessary. Thus, the need for drains and a continuous source of purified water are eliminated, rendering the system portable.

One of the drawbacks of sorbent dialysis systems is the high cost. The materials used in the sorbent cartridges can be expensive. Disposing of the cartridges after each use generates waste and drives up costs. Other known dialysate fluid circulation systems and apparatuses have separate housings where a first housing has a material capable of releasing sodium into dialysate fluid flowing through the first housing, and a second housing has a material capable of binding sodium ions from dialysate fluid flowing through the second housing. However, such systems cannot be formed into a single housing design, oftentimes require many liters of water, and may not be portable. The systems also do not provide for recharging some or all of the components of a sorbent cartridge that would allow reuse of specific components and enable lower long-term costs for operating such systems. Sorbent cartridges are known from US 2013/213890 and US 2011/048949.

Hence, there is a need for a sorbent cartridge having a separation of materials within the sorbent cartridge into modules to allow for isolation of those materials. There is a need for a sorbent cartridge providing for isolation of one or more sorbent materials to allow for cheaper or non-reusable materials to be discarded, while more expensive and reusable materials are recharged. There is a further need for a unitary sorbent cartridge having multiple discreet modules that can be easily connected and/or detached from the unitary sorbent cartridge thereby facilitating the recharging and/or recycling of the sorbent materials and the sorbent cartridge while retaining a single unitary design. There is also a need for a modular sorbent cartridge having the features of reduced size and weight necessary for a portable dialysis machine. There is a need for a modular sorbent cartridge wherein the sorbent materials can be arranged within the modules of the cartridge to allow for isolation of particular materials or groups of materials. There is further a need for any one of the modules in the cartridge to be reusable or optionally detachable from the cartridge to allow any one of disposal, recycling or recharging of sorbent material within the module. There is a need for a sorbent cartridge having specific materials that can be recharged and allowing for disposal of less expensive materials.

There is a need for the sorbent materials to be recharged without removing the modules from the sorbent cartridge during operation, making the system easier to use. There is a need for a recharging means directly attached to the sorbent modules, to allow the modules to be recharged simply by directing fluid flow from the rechargers to the module. There is further a need for one or more of the modules to be removable to allow for the recycling and/or disposal of these modules, while allowing for the recharging of other modules.

### SUMMARY OF THE INVENTION

The first aspect of the invention relates to a sorbent cartridge as defined by claim 1.

The second aspect of the invention relates to a method of recharging a sorbent as defined by claim 7.

### BRIEF DESCRIPTION OF THE DRAWINGS

FIG. 1 shows a sorbent cartridge containing activated carbon, hydrous zirconium oxide, urease, alumina, and zirconium phosphate.
FIG. 2 shows a modular sorbent cartridge with two modules.
FIG. 3 shows a modular sorbent cartridge with two modules including activated carbon, alumina, urease, and zirconium oxide in the first module, which can be a reusable module, and zirconium phosphate in the second module, which can be a reusable module.
FIG. 4 shows a method for recharging the zirconium phosphate sorbent material.
FIG. 5 shows a modular sorbent cartridge with two modules including activated carbon, zirconium phosphate, urease, alumina, and hydrous zirconium oxide in the first module, , which can be a reusable module, and zirconium phosphate in the second module.
FIG. 6 shows a modular sorbent cartridge with two modules including activated carbon, ion exchange resin, alumina, urease, and hydrous zirconium oxide in the first module, which can be a reusable module, and zirconium phosphate in the second module.
FIG. 7 shows a modular sorbent cartridge with two modules including activated carbon, alumina, urease, and zirconium phosphate in the first module, which can be a reusable module, and hydrous zirconium oxide and zirconium phosphate in the second module.
FIG. 8 shows a modular sorbent cartridge with two modules including activated carbon, alumina, urease, and hydrous zirconium oxide in the first module, which can be a reusable module, and ion exchange resin and zirconium phosphate in the second module.
FIG. 9 shows a modular sorbent cartridge with two modules including activated carbon, alumina, and urease in the first module, which can be a reusable module, and hydrous zirconium oxide, ion exchange resin, and zirconium phosphate in the second module.
FIG. 10 shows a modular sorbent cartridge with two modules including sodium chloride/sodium bicarbonate, activated carbon, ion exchange resin, active jack bean meal (JBM)/alumina, alumina, hydrous zirconium oxide/glass beads, and sodium chloride in the first module, which can be a reusable module, and zirconium phosphate in the second module.
FIG. 11 shows a modular sorbent cartridge with three modules including activated carbon, alumina, urease, and hydrous zirconium oxide in the first module, which can be a reusable module, zirconium phosphate in the second module, and zirconium phosphate and activated carbon in the third module.
FIG. 12 shows a modular sorbent cartridge with three modules including activated carbon in the first module, which can be a reusable module,, alumina and urease in the second module, and ion-exchange resin, zirconium phosphate, and hydrous zirconium oxide in the third module, with an optional bypass line to direct fluid from the first module, which can be a reusable module, to the third module.
FIG. 13 shows a modular sorbent cartridge with three modules and with an optional bypass line connected to another component such as a recharger.
FIG. 14 shows a modular sorbent cartridge with two modules and two connected bypass lines to direct flow around either module.
FIG. 15 shows a modular sorbent cartridge with two modules and a single bypass line to direct flow around either module.
FIG. 16 shows a modular sorbent cartridge with two modules and two separate bypass lines to direct flow around either module.
FIG. 17 shows a modular sorbent cartridge with two modules, two bypass lines and two rechargers.
FIG. 18 shows a modular sorbent cartridge with two modules, a single bypass line to bypass either module, or two rechargers.
FIG. 19 shows a modular sorbent cartridge with three modules, three bypass lines and three rechargers.
FIG. 20 shows a modular sorbent cartridge with two modules and two bypass lines to bypass the module, where the first module, which can be a reusable module, contains activated carbon, alumina, urease and hydrous zirconium oxide and the second module contains zirconium phosphate.
FIG. 21 shows a modular sorbent cartridge with three modules, three bypass lines, and three rechargers, wherein the first module, which can be a reusable module, contains activated carbon, the second module contains alumina and urease, and the third module contains hydrous zirconium oxide, ion-exchange resin and zirconium phosphate.
FIG. 22 shows a single module from a modular sorbent cartridge with a bypass line, and two wash lines each divided into gas and fluid wash lines.
FIG. 23 shows a single module from a modular sorbent cartridge with a bypass line, a recharger, and two wash lines each divided into gas and fluid wash lines.
FIG. 24 shows a controlled compliant dialysis circuit utilizing a sorbent cartridge.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

The term "before," as used when referring to relative positions of components of a dialysis system, refers to an upstream position in the normal operational flow direction of the dialysis system. The term "after" refers to a downstream position in the normal operational flow direction of the dialysis system.

"Blow out" refers to the process of passing a gas through a connection line or a module.

"Bypass line" refers to a line, connected to the main line, through which fluid or gas may alternatively flow.

The term "cartridge" refers to any container designed to contain a powder, liquid, or gas made for ready connection to a device, structure, system, flow path, or mechanism. The container can have one or more compartments. Instead of compartments, the container can also be comprised of a system of two or more modules connected together to form the cartridge wherein the two or more modules once formed can be connected to a device, structure, system, flow path, or mechanism.

The term "cation concentrate reservoir" refers to an object having or holding a substance that is comprised of at least one cation, for example calcium, magnesium, or potassium ions.

The term "cation infusate source" refers to a source from which cations can be obtained. Examples of cations include, but are not limited to, calcium, magnesium and potassium. The source can be a solution containing cations or a dry composition that is hydrated by the system. The cation infusate source is not limited to cations and may optionally include other substances to be infused into a dialysate or replacement fluid, non-limiting examples can be glucose, dextrose, acetic acid and citric acid.

The term "comprising" includes, but is not limited to, whatever follows the word "comprising." Thus, use of the term indicates that the listed elements are required or mandatory but that other elements are optional and may or may not be present.

A "connector" as used herein forms a fluid connection between two components wherein fluid or gas can flow from one component, through the connector, to another component. The connector provides for a fluid connection in its broadest sense and can include any type of tubing, fluid or gas passageway, or conduit between any one or more components of the invention.

The term "consisting of' includes and is limited to whatever follows the phrase "consisting of." Thus, the phrase indicates that the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "container" as used herein is a receptacle that may be flexible or inflexible for holding any fluid or solid, such as for example a spent dialysate fluid, or a sodium chloride or sodium bicarbonate solution or solid, or urease, or urease/alumina, or the like.

The terms "controlled compliance" and "controlled compliant" describe the ability to actively control the transfer of fluid volume into or out of a compartment, flow path or circuit. In any embodiment of the invention, the variable volume of fluid in a dialysate circuit or controlled compliant flow path expands and contracts via the control of one or more pumps in conjunction with one or more reservoirs. The volume of fluid in the system is generally constant (unless additional fluids are added to a reservoir from outside of the system) once the system is in operation if patient fluid volume(s), flow paths, and reservoirs are considered part of the total volume of the system (each individual volume may sometimes be referred to as a fluid compartment). The attached reservoirs allow the system to adjust the patient fluid volume by withdrawing fluid and storing the desired amount in an attached control reservoir and/or by providing purified and/or rebalanced fluids to the patient and optionally removing waste products. The terms "controlled compliance" and "controlled compliant" are not to be confused with the term "non-compliant volume," which simply refers to a vessel, conduit, container, flow path, conditioning flow path or cartridge that resists the introduction of a volume of fluid after air has been removed from a defined space such as a vessel, conduit, container, flow path, conditioning flow path or cartridge. In any embodiment of the invention, the controlled compliant system can move fluids bi-directionally. In certain cases, the bi-directional fluid movement can be across a semi-permeable membrane either inside or outside a dialyzer. The bi-directional fluid flow can also occur across, through, or between vessels, conduits, containers, flow paths, conditioning flow paths or cartridges of the invention in selected modes of operation. The term "moving fluid bi-directionally" as used in connection with a barrier, such as a semi-permeable membrane, refers to the ability to move fluid across the barrier in either direction. "Moving fluid bi-directionally" also can apply to the ability to move fluid in both directions in the flow path or between a flow path and reservoir in a controlled compliant system.

The terms "controlled compliant flow path," "controlled compliant dialysate flow path" and "controlled compliant solution flow path" refer to flow paths operating within a controlled compliant system having the characteristic of controlled compliance, or of being controlled compliant as defined herein.

A "control pump" is means capable of moving fluid through a system at a specific rate. The term "control pump," can include for example an "ultrafiltrate pump," which is a pump that is operable to pump fluid bi-directionally to actively control the transfer of fluid volume into or out of a compartment or circuit.

A "control system" consists of combinations of components that act together to maintain a system to a desired set of performance specifications. The control system can use processors, memory and computer components configured to interoperate to maintain the desired performance specifications. The control system can also include fluid or gas control components and solute control components as known within the art to maintain the performance specifications.

A "control valve" is a valve for controlling the movement of a liquid or a gas. When the control valve directs the movement of gas, the "control valve" can open or close to regulate the movement of gas from a high pressure gas source to a lower pressure.

A "controller," "control unit," "processor," or "microprocessor" is a device which monitors and affects the operational conditions of a given system. The operational conditions are typically referred to as output variables of the system wherein the output variables can be affected by adjusting certain input variables.

A "degasser" is a component that is capable of removing dissolved and undissolved gasses from fluids.

The term "detachable" or "detached" relate to any component of the present invention that can be separated from a system, module, cartridge or any component of the invention. "Detachable" can also refer to a component that can be taken out of a larger system with minimal time or effort. In certain instances, the components can be detached with minimal time or effort, but in other instances can require additional effort. The detached component can be optionally reattached to the system, module, cartridge or other component. A detachable module can often be part of a reusable module.

"Dialysate" is the fluid that passes through the dialyzer on the side of the dialysis membrane that is opposite to the fluid (e.g. blood) that is being dialyzed..

"Dialysis" is a type of filtration, or a process of selective diffusion through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids. The dialysate is used to remove solutes from the fluid to be dialyzed. The dialysate can also provide enrichment to the other fluid.

The term "dialyzer" refers to a cartridge or container with two flow paths separated by semi-permeable membranes. One flow path is for blood and one flow path is for dialysate. The membranes can be in the form of hollow fibers, flat sheets, or spiral wound or other conventional forms known to those of skill in the art. Membranes can be selected from the following materials of polysulfone, polyethersulfone, poly(methyl methacrylate), modified cellulose, or other materials known to those skilled in the art.

"Disposable" refers to a component that is to be removed from the system and not reused.

The term "extracorporeal," as used herein generally means situated or occurring outside the body.

The term "extracorporeal circuit" or "extracorporeal flow path" refers to a fluid pathway incorporating one or more components such as but not limited to conduits, valves, pumps, fluid connection ports or sensing devices configured therein such that the pathway conveys blood from a subject to an apparatus for hemodialysis, hemofiltration, hemodiafiltration or ultrafiltration and back to the subject.

The terms "extracorporeal flow path pump" and "blood pump" refer to a device to move or convey fluid through an extracorporeal circuit. The pump may be of any type suitable for pumping blood, including those known to persons of skill in the art, for example peristaltic pumps, tubing pumps, diaphragm pumps, centrifugal pumps, and shuttle pumps.

"Flow" refers to the movement of a fluid, gas, or both.

A "flow sensing apparatus" or "flow measuring apparatus" is an apparatus capable of measuring the flow of liquid or gas within a specific area.

A "fluid" is a is a subset of the phases of matter and can include liquids, gases, plasmas and, to some extent, plastic solids. Notably, a liquid, as used herein, can therefore also have a mixture of gas and liquid phases of matter.

The term "fluid communication" refers to the ability of fluid or gas to move from one component or compartment to another within a system or the state of being connected, such that fluid or gas can move by pressure differences from one portion that is connected to another portion.

The term "fluidly connectable" refers to the ability of providing for the passage of fluid or gas from one point to another point. The two points can be within or between any one or more of compartments, modules, systems, components, and rechargers, all of any type.

"Infusate" is a solution of one or more salts for the adjustment of the composition of a dialysate.

The term "in-line" refers to a state in which a module or set of modules is fluidly connected to a dialysis machine, dialysis flow path or dialysis circuit. Dialysis can be on-going, paused or stopped during the in-line state wherein in-line only refers to the state of the modules being fluidly connected to the dialysis machine, dialysis flow path or dialysis circuit.

"Module" refers to a discreet component of a system. Each of the modules can be fitted to each other to form a system of two or more modules. Once fitted together, the modules can be in fluid connection and resist inadvertent disconnection. A single module can represent a cartridge to be fitted to a device or mechanism if the module is designed to contain all the necessary components for an intended purpose such as a sorbent for use in dialysis. In such a case, the module can be comprised of one or more compartments within the module. Alternatively, two or more modules can form a cartridge to be fitted to a device or mechanism where each module individually carries separate components but only when connected together contain in summation all the necessary components for an intended purpose such as a sorbent for use in dialysis. A module can be referred to as a "first module," "second module," "third module," *etc.* to refer to any number of modules. The designation of "first," "second," "third," etc. does not refer to the respective placement of the module in the direction of fluid or gas flow, and merely serves to distinguish one module from another unless otherwise indicated.

The term "non-reusable" refers to a component that cannot be reused in the component's current state. In certain instances, the term non-reusable can include the concept of being disposable, but is not necessarily limited to just being disposable.

The term "off-line" refers to a state in which a module or set of modules is fluidly disconnected from a dialysis machine, dialysis flow path or dialysis circuit. Dialysis can be on-going, paused or stopped during the off-line state wherein off-line only refers to the state of the modules being fluidly disconnected from the dialysis machine, dialysis flow path or dialysis circuit. The off-line state can also include a process whereby the module or set of modules is being recharged as defined herein.

An "operational line" or "line" is a passageway, conduit or connector that directs fluid or gas in a path used while the system is in operation.

The terms "pathway," "conveyance pathway," "fluid flow path," and "flow path" refer to the route through which a fluid or gas, such as dialysate or blood travels.

A "photocell" is a sensor capable of measuring light or other electromagnetic radiation.

The terms "pressure meter" and "pressure sensor" refer to a device for measuring the pressure of a gas or liquid in a vessel or container.

A "pressure valve" is a valve wherein, if the pressure of the fluid or gas passing the valve reaches a certain level, the valve will open to allow fluid or gas to pass through.

The term "pump" refers to any device that causes the movement of fluids or gases by the application of suction or pressure.

A "push-on fitting" is a fitting for connecting two components wherein the components may be connected by applying pressure to the base of the fitting attached to the components.

A "quick connect fitting" is a fitting for connecting two components wherein the male portion of the fitting contains flexible flanges extending outward with a portion on the end of the flange extending further outward, and the female portion of the fitting contains an internal ridge so that when connected, the outward extending portion of the flange sits under the ridge. By applying pressure, the flexible flange can be forced inward, past the ridge, enabling easy removal.

A "recharger" is a component that is capable of recharging spent sorbent material to or near its original state. A recharger may be part of the dialysis system or may be separate from the rest of the system. If the recharger is separate from the rest of the dialysis system, the term may include a separate facility where the spent sorbent material is sent to be returned to, or near, its original state. A "recharger connector" or "recharger node" is a connector that fluidly connects a recharger to another component.

"Recharging" refers to the process of treating spent sorbent material to restore the functional capacity of the sorbent material so as to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

"Reusable" refers in one instance to a material that can be used more than one time, optionally with treatment of any type of the material between uses. For example, a material and a solution can be reused. In one instance, reusable can refer to a cartridge, as used herein, that contains a material that can be recharged by recharging the material(s) contained within the cartridge.

A "sensor" is a component capable of determining the states of one or more variables in a system.

"Sorbent cartridge" refers to a cartridge that can contain one or more sorbent materials. The cartridge can be connected to a dialysis flow path. The sorbent materials in the sorbent cartridge are used for removing specific solutes from solution, such as urea. The sorbent cartridge can have a single compartmental design wherein all sorbent materials necessary for performing dialysis are contained within the single compartment. Alternatively, the sorbent cartridge can have a modular design wherein the sorbent materials are dispersed across at least two different modules, which can be connected to form a unitary body. Once the at least two modules are connected together, the connected modules can be referred to as a sorbent cartridge, which can be fitted to a device or mechanism. It will be understood that when a single module contains all the sorbent materials necessary for performing dialysis, the single module can be referred to as a sorbent cartridge.

"Sorbent materials" are materials capable of removing specific solutes from solution, such as urea.

"Spent dialysate" is a dialysate contacted with blood through a dialysis membrane and contains one or more impurity, or waste species, or waste substance, such as urea.

The term "substantially inflexible volume" refers to a three-dimensional space within a vessel or container that can accommodate a maximum amount of non-compressible fluid and resists the addition of any volume of fluid above the maximum amount. The presence of a volume of fluid less than the maximum amount will fail to completely fill the vessel or container. Once a substantially inflexible volume has been filled with a fluid, removal of fluid from that volume will create a negative pressure that resists fluid removal unless fluid is added and removed simultaneously at substantially equal rates. Those skilled in the art will recognize that a minimal amount of expansion or contraction of the vessel or container can occur in a substantially inflexible volume; however, addition or subtraction of a significant volume of fluid over the maximum or minimum will be resisted.

"Tap water" refers to water obtained through piping from a water supply without additional treatment.

A "threaded fitting" is a fitting for connecting two components wherein the male portion has a helical ridge wrapped around a cylinder, and the female portion is a cylindrical hole with internal helical ridges so that when the male portion is screwed into the female portion the two components are locked together.

A "twist-lock fitting" is a fitting for connecting two components wherein the male portion of the fitting contains a head with a length exceeding its width, the female portion of the fitting is a hole with a length that exceeds its width and is larger than the male portion, so that when the male portion is inserted into the female portion and either portion is twisted the two components become locked together.

"Uremic toxins" are toxins carried in the blood supply normally removed in the kidneys.

A "valve" is a device capable of directing the flow of fluid or gas by opening, closing or obstructing one or more pathways to allow the fluid or gas to travel in a particular path. One or more valves configured to accomplish a desired flow can be configured into a "valve assembly."

A "wash line" is a line that directs fluid between a recharger and a module.

The term "waste fluid" refers to any fluid that does not have a present use in the operation of the system. Non-limiting examples of waste fluids include ultrafiltrate, or fluid volume that has been removed from a subject undergoing a treatment, and fluids that are drained or flushed from a reservoir, conduit or component of the system.

The term "water source" refers to a source from which potable or not potable water can be obtained.

The term "waste species," "waste products," "waste," or "impurity species" refer to any molecular or ionic species originating from the patient or subject, including metabolic wastes, molecular or ionic species including nitrogen or sulfur atoms, mid-weight uremic wastes and nitrogenous waste. Waste species are kept within a specific homeostasis range by individuals with a healthy renal system.

### Sorbent Dialysis

Sorbent dialysis allows dialysis with a small volume of dialysate, creating many advantages. In sorbent dialysis, spent dialysate, containing toxins removed from the blood of the patient, is passed through a sorbent cartridge. The sorbent cartridge of the invention can contain sorbent materials that selectively remove specific toxins from the spent dialysate, either completely or by replacing them with non-toxic material. This process converts the spent dialysate into clean dialysate, which is then redirected back to the dialyzer.

Modular sorbent cartridges, wherein each module contains select sorbent materials, can be useful in sorbent dialysis. This modular design critically allows for certain portions of the sorbent cartridge to be discarded, refilled, recycled or recharged. In any embodiment of the invention, the sorbent materials can be structured into layers and/or intermixed. In particular, the modules can have the sorbent materials either intermixed or in layers wherein any combination of intermixed and layered modules can be used interchangeably together.

To save costs and waste, the modules of the modular sorbent cartridge may be rechargeable. The sorbent cartridges can be reusable or non-reusable, unless specifically specified as reusable. The sorbent material within the module can be recharged and made reusable by passing a solution containing the proper solutes through the layers of the sorbent module.

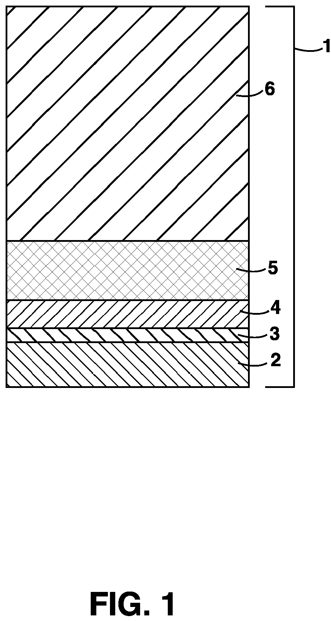

One non-limiting exemplary sorbent cartridge is shown in FIG. 1. Spent dialysate or fluid can flow from the bottom of the sorbent cartridge **1** to the top of the cartridge. The first sorbent material the spent dialysate (or fluid) contacts can be activated carbon **2.** Activated carbon will remove nonionic toxins from the fluid by adsorption. Creatinine, glucose, uric acid, P2-microglobulin and other non-ionic toxins, except urea, can be adsorbed onto the activated carbon, removing those toxins from the fluid. Other non-ionic toxins will also be removed by the activated carbon. The dialysate (or fluid) then continues through the sorbent cartridge to the hydrous zirconium oxide layer **3.** The hydrous zirconium oxide layer **3** can remove phosphate and fluoride anions, exchanging them for acetate anions. The fluid can continue to move through the sorbent cartridge into the alumina/urease layer **4.** Urease can catalyze the reaction of urea to form ammonia and carbon dioxide. The result of this is the formation of ammonium carbonate. The phosphate anions present in the fluid can also be exchanged for hydroxide ions on the alumina. As the fluid continues through the sorbent cartridge, it reaches alumina layer **5.** The alumina layer **5** can remove any remaining phosphate ions from the fluid and help retain urease within the sorbent cartridge, and in certain configurations this layer can exchange urea for ammonium and other components. The last layer through which the fluid travels can be the zirconium phosphate layer **6.** In the zirconium phosphate layer **6,** ammonium, calcium, potassium and magnesium cations can be exchanged for sodium and hydrogen cations. Ammonium, calcium, potassium and magnesium ions all preferentially bind to the zirconium phosphate, releasing the hydrogen and sodium ions originally present in the zirconium phosphate layer **6.** The ratio of sodium to hydrogen ions released depends on the ratio originally present in the zirconium phosphate layer **6,** and is therefore controllable. The result of the fluid passing through the sorbent cartridge **1** is that the fluid can be regenerated and form clean dialysate that can be safely passed back through a dialyzer to a patient. In any embodiment of the invention, potassium, calcium, and magnesium can be added to the clean dialysate to replace any ions which were removed by the sorbent cartridge. The ions can be added and/or controlled via an infusate system that can be positioned on a section of the fluid flow path after the sorbent cartridge.

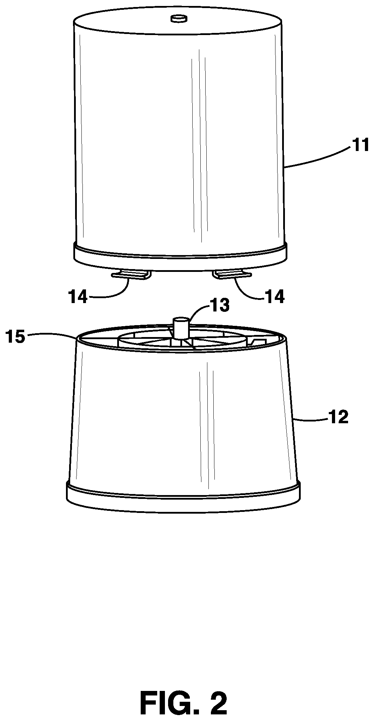

Given the cost of the sorbent cartridges and sorbent materials, it would be advantageous if parts of the cartridge could be reused or recharged. The present invention relates to a sorbent cartridge which includes at least one reusable module. As shown in FIG. 2, a reusable module **11** can be fluidly attached to a non-reusable module **12** by a connector **13** with the use of latches **14** disposed near the circumference of the reusable module **11.** The latches **14** can be integrally formed as part of the reusable module **11,** or non-reusable module **12.** Alternatively, they may be a separate component that must be attached to the module **11.** The latch members **14** can be mated to an annular connection ring **15** disposed on the circumference of module **12.** One or more engagement members can be disposed inside the annular connection ring **15** to engage the latches **14** when positioned relative to each other using a radial motion. Such engagement can cause a rigid connection between the reusable module **11** and the non-reusable module **12.** Other known locking or fastening mechanisms known to those of ordinary skill that can effectuate rapid and effective connections between two components are contemplated by the invention. Although only cylindrical modules are shown, it will be understood that modules of any shape such as rectangular, conical, triangular, etc. are contemplated by the present invention with a correspondent fastening mechanism. In any embodiment of the invention, the connector **13** can be formed as part of the reusable module **11** and non-reusable module **12** and need not be a separate component that must be attached to the module **12.** Rather, the connector **13** can be molded as part of the reusable module **11** and non-reusable module **12.** The connector can be a combination of female and male connectors on a module. For example, a female connector can be disposed on one module, and a male connector on the other to form one connector **13** (not shown). In any embodiment of the invention, the connector can be affixed by mechanical means, glued or rigidly interfaced to the modules **11** and **12.** In any embodiment, the connector **13** allows fluid to flow from the non-reusable module **12,** through the connector **13,** into the reusable module **11.** Alternatively, in any embodiment of the invention, the connector **13** is not a part of either the non-reusable module **12** or reusable module **11** but can be a separate component such as tubing. It will be understood that the connector **13** is defined in its broadest sense and encompasses any fluid connection between two points.

It will be understood that different combinations of reusable and non-reusable modules can be combined together. Moreover, any one of the modules can be detachable from each other or from a casing forming the body of the sorbent cartridge. The modules can be standardized components that are interchangeable with other modules and easily assembled. For example, the latches **14** in FIG. 2 allow for a simple, twist-lock between two modules. The twist lock allows for the modules to be connected to each other by an easy and rapid manual motion not requiring complex maneuvering of the modules. The connection, once made, can be resistant to inadvertent disengagement, but can also be readily disengaged when desired with a similar easy and rapid manual manipulation. For example, a force applied on the outside periphery of the modules near the latch, e.g. squeezing the module, can cause the latch member **14** to disengage from the engagement members. In other examples. The modules can be disengaged by simply rotation the modules relative to each other.

At least two modules can cooperate together when engaged to each other using, for example, the latches **14** in FIG. 2 and being fluidly connected together to function as a sorbent cartridge. The advantage of such a modular design as described herein is that different sorbent materials can be dispersed between the at least two modules to allow for any particular sorbent or combination of sorbent materials to be detachable from a sorbent cartridge.

In certain embodiments, the connector **13** can be formed as part of the reusable module **11** and non-reusable module **12** and need not be a separate component that must be attached to the module **12.** Rather, the connector **13** can be molded as part of the reusable module **11** and non-reusable module **12.** The connector can be a combination of female and male connectors on a module. For example, a female connector can be disposed on one module, and a male connector on the other to form one connector **13** (not shown). In any embodiment of the invention, the connector can be affixed by mechanical means, glued or rigidly interfaced to the modules **11** and **12.** In any embodiment of the invention, the connector **13** allows fluid to flow from the non-reusable module **12,** through the connector **13,** into the reusable module **11.** Alternatively, the connector **13** is not a part of either the non-reusable module **12** or reusable module **11** but can be a separate component such as tubing. It will be understood that the connector **13** is defined in its broadest sense and encompasses any fluid connection between two points.

In any embodiment of the invention, one or more fluid connectors can be arranged between any module of the invention, and one or more such fluid connectors can be provided in any of the described configurations herein. For example, a reusable or non-reusable module can have any number of connectors such as 1, 2, 3, 4, 5, or more. The spacing and distribution of the fluid connectors on the module can be positioned to enable and or increase flow of fluid between the modules. In one example, the fluid connectors can be spaced equidistant from each other or may be located axially or radially. The sorbent cartridge can also have one or more modules each having any number of fluid connectors. In contrast to known sorbent cartridges having a unitary design in which sorbent materials are arranged in layers without any connectors between such layers, the fluid connectors of the present invention allow for controlled fluid or gas flow to any particular sorbent or combination of sorbent materials. The fluid connectors also allow for any particular sorbent or combination of sorbent materials to be detachable from a sorbent cartridge. For example, a detachable module can be constructed with one or more sorbent materials. The detachable module can then be fluidly connected to the sorbent cartridge by fluid connectors. Such a configuration advantageously allows for separate treatment, recycling, or recharging of the sorbent or combination or mixture of sorbent materials not possible with known sorbent cartridges. In particular, known sorbent cartridges have all the sorbent materials being formed into layers or a plurality of sorbent materials being mixed without connectors in between such layers of one sorbent material, or mixtures of sorbent materials. It will be understood that the fluid connectors of the invention can be critical because the connectors control the order of sorbent materials to which a fluid or gas is exposed, the delivery of fluid or gas to a particular sorbent or combination of sorbent materials, and the flow and rate of flow of a fluid or gas to various sorbent materials, layers of sorbent materials, and combination or mixtures of sorbent material.

In one aspect of the invention, it will be understood that the present invention contemplates at least two modules that fit together, which is distinct from known dialysis systems having separate housings that do not form a unitary sorbent cartridge for ready attachment or insertion into a dialysis machine. A unitary sorbent cartridge of the present invention contains one or more of the sorbent materials described herein. In any embodiment of the invention, the cation and anion exchange materials necessarily reside in the sorbent cartridge. In other words, the cation and anion exchange resins (or other sorbent materials) are not separated into different housings outside a sorbent cartridge. Although the individual sorbent materials of the present invention may be separated into different detachable and/or reusable modules within the single sorbent cartridge wherein each module is connected by fluid connectors, the single sorbent cartridge design provides reduced size and weight that is not possible with the known dialysis systems having separate housings. The modules, as described herein, can also be further rigidly fixed to each other by latches and engagement members or any fixing or fastening mechanism known to those of ordinary skill in the art. Notably, the sorbent cartridge of the present invention can have all of the sorbent materials described herein including cation and anion exchange resins within a single unitary sorbent cartridge for convenient removal, service and monitoring. In particular, the sorbent cartridge can have a single compartmental design wherein all sorbent materials necessary for performing dialysis are contained within a single compartment. The sorbent cartridge can also have a modular design wherein the sorbent materials are dispersed across at least two different modules, which can be connected to form a unitary body. Once the at least two modules are connected together, the connected modules can form a sorbent cartridge to be fitted to a device or mechanism. Advantageously, the present sorbent cartridge can therefore be easier to recycle, recharge, dispose of, service and remove from a dialysis machine. In any embodiment of the invention, the unitary design can also provide for a compact design that can be used in a portable dialysis machine. Further, manufacturability is benefited by the unitary design.

In any embodiment of the invention, the fluid connector can be a quick-connect, twist-lock fitting, push-on fitting, or threaded fitting. Other forms of such connection known to those of ordinary skill in the art are also contemplated by the present invention. Additionally, the connector can comprise a length of tubing and valve or a valve assembly. In any embodiment of the invention, the connector can be manually assembled to connect any component or assembly of the invention. The connector can also be used to rigidly connect any one of the modules to a recharger as defined herein when a separate fastening mechanism is not provided.

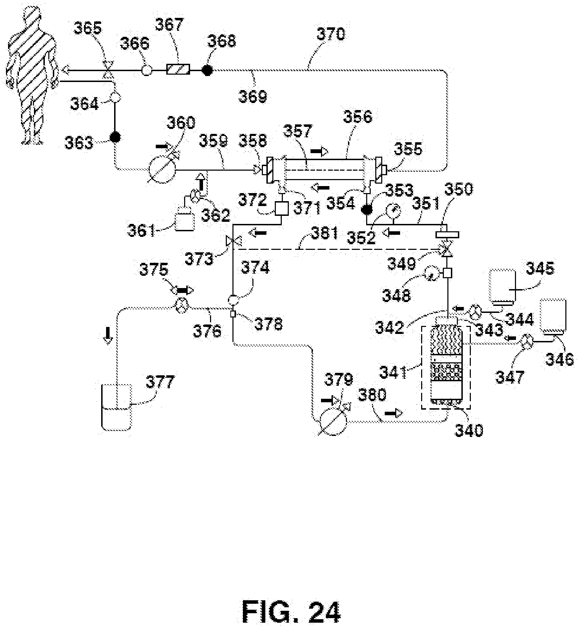

In any embodiment of the invention, at least one module can be in fluid communication with a controlled compliant dialysis circuit. A non-limiting example of a controlled compliant dialysis circuit is shown in FIG. 24. The patient's blood is circulated through an extracorporeal circuit **370.** The portion of the extracorporeal circuit **370** that contains blood drawn from the patient can be referred to as the arterial line **359,** which by convention is understood to mean a line for transporting blood from the patient regardless of whether blood is drawn from an artery or vein of the patient. Similarly, the portion that returns blood to the patient can be referred to as the venous line **369.** In any embodiment of the invention, the arterial line **359** and the venous line **369** connect with one or more veins of the patient. Locomotive power for moving the blood through the extracorporeal circuit **370** is provided by a blood pump **360,** which is typically located along the arterial line **359.** Valve **365** can be placed on venous line **369.** Blood is typically conveyed through the extracorporeal circuit **370** at a rate of 50 to 600 mL/min and can be adjusted by a controller to any required rate suitable for a procedure performed by the invention. Blood pump **360** can be a peristaltic pump, although those skilled in the art will readily understand that other types of pumps can be used including diaphragm pumps, centrifugal pumps, and shuttle pumps. In any embodiment of the invention, the blood pump **360** conveys blood through the dialyzer **356** where the blood is contacted with a blood side of a high permeability dialysis membrane **357.** Blood enters the dialyzer **356** through a blood inlet **358** and exits through a blood outlet **355.** The pressure of the blood prior to the blood pump **360** is measured by a pressure meter **363** and post dialyzer **356** by a pressure meter **368.** The pressure at pressure meter **363** provides an indication of the adequacy of the blood flow into the circuit where increased vacuum is an indication of a less adequate access flow. The pressure indication at pressure meter **368** can serve to detect obstructions in the venous bloodline. Additional pressure meter **353** can be located after blood outlet **355.** An air trap **367** is placed along the extracorporeal circuit **370** to prevent the introduction of air into the circulatory system of the patient. The air trap **367** is not limited to a particular design. Typical air traps employ a hydrophobic membrane that allows air to be separated from an air- liquid mixture by allowing air to pass through the membrane and retaining water-based fluids. Alternatively, in any embodiment of the invention, the air trap **367** can be run full, where a pressure meter can use a flexible impermeable membrane to transmit pressure pulses to a pressure transducer such that there is no direct air blood interface. Air-fluid detectors **364** and **366** are present to confirm that air is not present in the extracorporeal circuit **370,** and additional air-fluid detector **374** can be present in the dialysis circuit **380.** Air fluid detectors **364, 366** and **374** can be ultrasonic sensors that can detect a change in solution density or scattering due the presence of air or air bubbles.

During the course of conveyance of blood along the extracorporeal circuit **370,** heparin or other anticoagulant is added to the blood to prevent clotting of blood within the dialyzer **356** or blood conveyance pathway/extracorporeal circuit **370.** Heparin or another anticoagulant is added from an anticoagulant container **361** at a metered rate using an anticoagulant pump **362.** The anticoagulant pump **362** can be any pump capable of accurately metering heparin.

Dialysate within the system is conveyed through one of a first dialysate pathway **351** in the dialysate circuit, which carries dialysate to the dialyzer **356,** or a second bypass pathway **381** shown in a dashed line, which serves to bypass the dialyzer **356.** The dialysis circuit can include a pair of quick connectors **378.** The first and second pathways **351** and **351** have one or more conduits for conveying the dialysate. Access to the second bypass pathway **381** is controlled by valve **349.** It is understood by one skilled in the art that three-way valve **349** can be replaced with a two-way valve or four-way valve with the same result to control the flow through the dialyzer **356** or bypass pathway **381.** The first dialysate pathway **351,** the second bypass pathway **381,** and residual volume in the dialyzer **356** including conduits for conveying the dialysate together form a dialysis circuit **380** that houses the circulating volume of the dialysate present in the system. It is understood by one skilled in the art that three-way valve **349** could be replaced with two-way valves or four-way valves with the same result to control the flow through the dialyzer or bypass loop.

Dialysate that is conveyed through the dialyzer **356** on the dialysate side of the dialysis membrane **357** picks up waste products from the blood, including urea, by diffusion, hemofiltration or hemodiafiltration. Dialysate enters the dialyzer at a dialysate inlet end **354** and exits at an outlet end **371.** The dialysate exiting the dialyzer **356** passes through a blood leak detector **372** that can determine the presence of blood in the dialysate indicating a breach in the dialysis membrane **357.** Flow of dialysate from the dialyzer **356** can be stopped or controlled through the operation of valve **373** as well as to prevent the backup of dialysate into the dialyzer **356.** The dialysate is conveyed through a sorbent cartridge **341** to remove waste products before being re-conveyed through the dialyzer **356.** The dialysate enters the sorbent cartridge **341** at a dialysate inlet end **340** and exits at an outlet end **342.** Refreshed dialysate exiting an outlet end **342** of the sorbent cartridge **341** can be monitored by a conductivity meter **348.** Additional conductivity meter **352** can be present. Optionally, the dialysate can be filtered through a microbial filter **350.** An air trap **343** can be positioned before or after outlet end **342** to remove gasses introduced into the dialysate by the sorbent cartridge **341.** The volume of actively circulating dialysate is determined by the total void volume of the conduits and the sorbent cartridge **341** forming the dialysis circuit **380.** The void volumes of the conduits and of the sorbent cartridge **341** forming the dialysis circuit **380** have a non-expandable or substantially inflexible volume.

The total void volume of the conduits having a substantially inflexible volume prevents the passive inflow and outflow of fluid volume due to pressure changes that can occur over the course of treatment. This results in a benefit because not all of the pressure changes during treatment are under precise control by a user or operator. A controlled compliance dialysis circuit is achieved by actively controlling the inflow (influx) and outflow (efflux) of fluid to and from the dialysis circuit **380** and the extracorporeal circuit **370.** In this manner, the volume of fluid crossing the dialysate membrane **357** is under direct control and can be accurately determined.

The controlled compliance dialysis circuit can be accurately controlled to precisely remove or add fluid to the dialysis circuit. Due to the substantially inflexible void volume of the conduits, the sorbent cartridge **341** and other components of the dialysis circuit **380,** the net movement of fluid over any time interval across the dialysate membrane can be accurately controlled by creating a means to accurately introduce or remove fluid from the patient. This capability is used to enhance the convective clearance of the system while controlling the net fluid removed from the patient.

As shown in FIG. 24, the dialysate is moved along the dialysis circuit **380** by a dialysate pump **379.** When the control pump **375** is not operating, fluid along the length of the dialysis circuit **380** flows at a rate determined by the dialysate pump **379.** When the control pump **375** is operating, fluid exiting the dialyzer **356** and traveling toward the conduit **376** is flowing at a rate that is the combination of the rates of the control pump **375** and the dialysate pump **379.** However, the fluid traveling from the entry point of conduit **376** into the dialysis circuit **380** to the dialyzer **356** is traveling at the rate of the dialysate pump **379.** As such, the rate of fluid traveling to the dialyzer **356** is not affected by the operation of the control pump **375.** The dialysate pump can be operated at a rate from about 10 to about 400 mL/min, the specific rate being dependent on the rate of the blood pump **360** at the desired contact time with the dialysis membrane **357** to achieve diffusion of impurities from blood to the dialysate. The rate of the dialysate pump **379** and the blood pump **360** can be controlled by a controller (not shown).

Due to the substantially inflexible void volume of the conduits and the sorbent cartridge **341,** bulk fluid or water is prevented from moving across the membrane **357** from the extracorporeal circuit **370** of the dialyzer **356** to the dialysate circuit **380** of the dialyzer **356.** Specifically, due to the controlled compliant feature of the void volume of the dialysis circuit **380,** water cannot passively move from the extracorporeal side to the dialysate side through the dialysis membrane. In the event of factors that tend to increase pressure on the extracorporeal side of the dialysis membrane, such as increased blood flow rate or blood viscosity, pressure across the membrane will automatically be equalized due to the limited volume of the dialysis circuit **380** and the non-compressible nature of the dialysate. In the event of factors that tend to increase pressure on the dialysate side of the dialysis membrane **357,** such as increased dialysis flow rate, net movement of water from the dialysis circuit **380** to the extracorporeal circuit **370** is prevented by a vacuum that would form in the dialysate circuit **380** in the event of such a movement. Since the dialyzer can be a high flux type, there is some fluid flux back and forth across the dialyzer membrane due to the pressure differential on the blood and dialysate sides of the membrane. This is a localized phenomenon due to the low pressure required to move solution across the membrane and is called backfiltration, however results in no net fluid gain or loss by the patient.

Using the controlled compliance dialysis circuit described herein, net movement of water across the dialysis membrane occurs under active control rather than passively due to pressure differences that develop across the dialysis membrane due to normal operations. A control pump **375** is present and accesses the controlled compliance dialysis circuit **380** through a conduit **376.** In any embodiment of the invention, the conduit **376** joins with the controlled compliance dialysis circuit **380** at a point downstream from the dialyzer **356.** The control pump **375** can be operated in an influx direction that moves fluid from a control reservoir **377** to the controlled compliance dialysis circuit **380** or in an efflux direction that moves fluid from the controlled compliance dialysis circuit **380** into the control reservoir **377.** Due to the substantially inflexible volume of the dialysis circuit **380,** volume added to the controlled compliance dialysis circuit when the control pump **375** operates in the influx direction causes net movement of fluid from the dialysate side of the dialysis membrane **357** to the extracorporeal side of the dialysis membrane **357.** When the control pump .**375** is operated in the efflux direction, fluid is drawn from the extracorporeal side of the dialysis membrane **357** into the controlled compliance dialysis circuit. In any embodiment of the invention, the control pump **375** can be operated at a rate from 0 to about 500 mL/min in either direction.

An infusate pump **344** can be used to add a cation infusate **345** into the hemofiltration circuit **380** to generate a fluid having a proper physiological composition to serve as a replacement fluid for introduction into the extracorporeal circuit **370.** A bicarbonate solution in a container **346** can further be added by a pump **347** to maintain a physiological pH in the fluid prior to introduction to the extracorporeal circuit.

It will be understood that the connector provides for a fluid connection in its broadest sense and can include any type of tubing, fluid or gas passageway, or conduit between any one or more components of the invention.

The sorbent material within the module can be recharged by passing a solution containing the proper solutes through the layers of the sorbent module. To recharge the sorbent modules in-line, the modules may be connected by wash lines to rechargers, which contain solutions for recharging the modules.

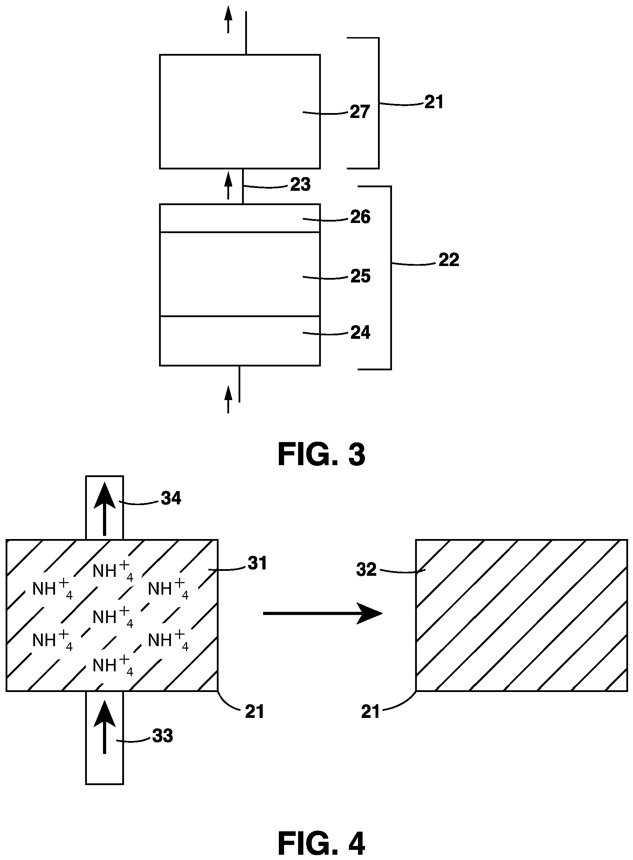

One embodiment of a modular sorbent cartridge of the invention is shown in FIG. 3. The non-reusable module **22** of the sorbent cartridge contains layers of activated carbon **24,** alumina/urease **25,** and hydrous zirconium oxide **26.** The reusable module **21** contains zirconium phosphate **27.** In any embodiment of the invention, the term non-reusable can refer to the components in a cartridge, and in any embodiment of the invention, the term can refer to both the components in the cartridge and the cartridge itself.

After dialysis is complete, the zirconium phosphate layer **27** can contain ammonium, calcium, potassium and magnesium. The module **21** containing the zirconium phosphate may be removed, and the zirconium phosphate recharged. The reusable module can be disconnected from the connectors **23** connecting the reusable module to the non-reusable module, bypass line and/or wash line. The reusable module **21** is then removed from the modular sorbent cartridge. In any embodiment of the invention, the module **21** can then be recharged, discarded and replaced, or alternatively, the sorbent material within the module can be removed and refilled. It will be understood that any one of the materials used in the present invention can be used multiple times. In such instances of multi-session use, the number of sessions in which one component can be used, can be the same or different from the number of sessions in which another component can be used. In one non-limiting example, a module containing urease may be used two times while another module containing zirconium phosphate can be used three times. In other cases, the module containing urease can be used three times, and the module containing zirconium phosphate used two times. It will be understood that there is no limitation on the numbers of uses for any multi-session use module as compared to another module used in the sorbent cartridge.

A method of recharging the zirconium phosphate module is shown in FIG. 4. Wash fluid **33,** containing sodium and hydrogen ions, can be passed through the reusable module **21,** containing the used zirconium phosphate **31** with bound ammonium ions. This causes an exchange of ions, wherein hydrogen and sodium ions can replace the ammonium ions on the zirconium phosphate **31.** The waste fluid **34** exiting the module **21** thus contains the freed ammonium ions, with excess sodium and hydrogen ions. This process creates a recharged zirconium phosphate layer **32,** containing sodium and hydrogen ions for a subsequent dialysis. In any embodiment of the invention, a recharger can be used to recharge spent sorbent material wherein the recharger contains fluid capable of restoring spent sorbent material to, or near, its original state or usable capacity.

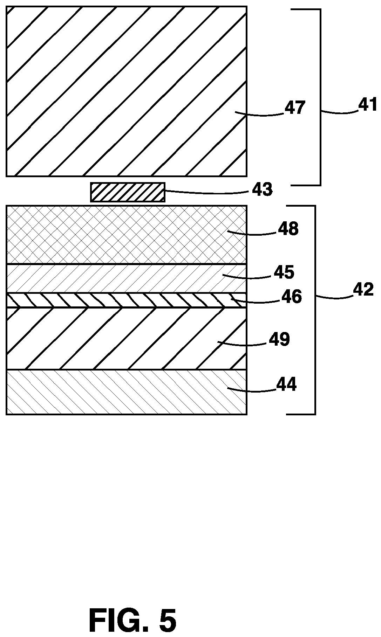

Because calcium and magnesium ions may be more difficult to remove from the zirconium phosphate, and therefore the zirconium phosphate may be more difficult to recharge, it may be advantageous to remove the calcium and magnesium in the first, non-reusable module, so that none of those ions need to be removed in the reusable zirconium phosphate module. Such an embodiment of the invention is in FIG. 5. Spent dialysate enters the first, non-reusable module **42** where the spent dialysate can first flow through a layer of activated carbon **44** to remove non-ionic uremic toxins. The spent dialysate can then enter into a first layer of zirconium phosphate **49.** The zirconium phosphate layer **49** can remove the calcium, magnesium and potassium from the fluid. Next the fluid can enter the hydrous zirconium oxide layer **46,** which can remove the phosphate anions and exchange them with acetate anions. The fluid can then enter the urease layer **45** and alumina layer **48,** where the urea can be converted to ammonium carbonate and any remaining phosphate ions can be removed. In any embodiment of the non-reusable module of the invention, any arrangement of the activated carbon, zirconium phosphate, hydrous zirconium oxide layer, and urease and alumina layer is contemplated. For example, the dialysate can first flow through a first layer of zirconium phosphate, activated carbon, then the hydrous zirconium oxide layer, and then enter the urease layer and alumina layer. Alternatively, in any embodiment of the invention, the dialysate can first flow through the hydrous zirconium oxide layer, then a first layer of zirconium phosphate, the activated carbon, then enter the urease layer and alumina layer. Still further, the dialysate can first flow through the urease layer and alumina layer, then the hydrous zirconium oxide layer, then a first layer of zirconium phosphate, and then the activated carbon. The fluid can then flow through the connector **43,** and into the second, reusable, sorbent module **41.** The second sorbent module **41** can contain zirconium phosphate **47.** Zirconium phosphate layer **47** can exchange the ammonium ions for sodium and hydrogen. Because the calcium, magnesium and potassium ions have already been removed by the first zirconium phosphate layer **49,** this second layer **47** will not pick up those ions. After dialysis, the second module **41** will only contain zirconium phosphate bound to ammonium ions. As such, the sorbent material may be easier to recharge.

In embodiments of the invention where the reusable module contains zirconium phosphate and ion-exchange resin, or zirconium phosphate and hydrous zirconium oxide, the module may be recharged in the same manner. The activated carbon layer of a reusable module may be recharged by passing a heated water solution through the activated carbon layer. The alumina/urease layers can be recharged by first passing heated water, or the solutions described above for recharging zirconium phosphate, through the layer, and then passing a solution containing urease through alumina/urease layers.

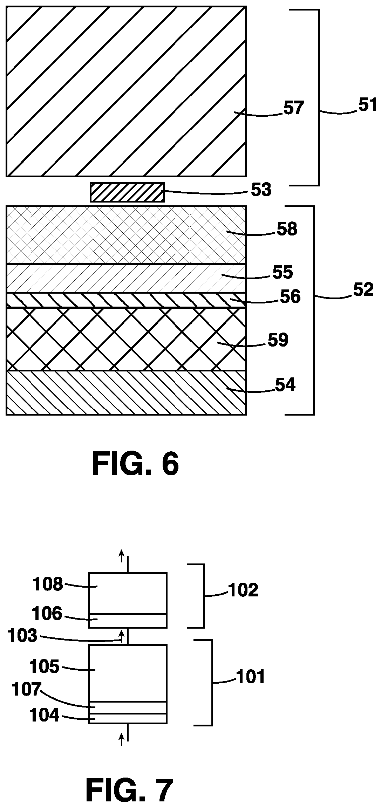

Another non-limiting embodiment of the invention is shown in FIG. 6. Spent dialysate can enter the first, non-reusable, module **52** where it first flows through a layer of activated carbon **54** to remove non-ionic uremic toxins. The spent dialysate then enters into a layer of ion exchange resin **59.** The ion-exchange resin layer **59** removes the calcium, magnesium and potassium from the fluid. Next the spent dialysate can enter the hydrous zirconium oxide layer **56,** which removes the phosphate anions and exchanges them with acetate anions. The spent dialysate then enters the urease layer **55** and alumina layer **58,** where the urea is converted to ammonium carbonate and any remaining phosphate ions are removed. In any embodiments of the first, non-reusable, module **52** of the invention, any arrangement of the activated carbon, ion exchange resin, hydrous zirconium oxide layer, and urease and alumina layer is contemplated. For example, the dialysate can first flow through an ion exchange resin, activated carbon, then the hydrous zirconium oxide layer, and then enter the urease layer and alumina layer. Alternatively, in any embodiment of the invention, the dialysate can first flow through the hydrous zirconium oxide layer, then the ion exchange resin, the activated carbon, then enter the urease layer and alumina layer. Still further, the dialysate can first flow through the urease layer and alumina layer, then the hydrous zirconium oxide layer, then the ion exchange resin, and then the activated carbon. The fluid can then flow through the connector **53,** and into the second, reusable, sorbent module **51.** The sorbent module **51** contains zirconium phosphate **57.** The zirconium phosphate layer **57** can exchange the ammonium ions for sodium and hydrogen. Because the calcium, magnesium and potassium ions have already been removed by the ion-exchange resin layer **59,** the zirconium phosphate layer **57** will not pick up those ions. Alternatively, in any embodiment of the invention, the ion-exchange resin **59** may be selected to only remove the calcium and magnesium ions, such as by using a chelating ion exchange resin. This will allow use of less of the ion exchange resin. If such a resin is used, the potassium will be removed by the zirconium phosphate **57.** Potassium is easier to remove from zirconium phosphate than calcium or magnesium. In any embodiment of the invention, the sorbent materials in each module may be intermixed as opposed to being arranged in layers.

One skilled in the art will recognize that different combinations of sorbent materials in both the reusable and non-reusable modules of the sorbent cartridge can be used without being beyond the scope of this invention. The sorbent materials described herein can be mixed together in any combination as shown in the specific embodiments of the invention.

In any embodiment of the invention, the sorbent cartridge can be removed from a dialysis system. The sorbent cartridge once removed can be separated into one or more modules to be recharged, disposed of, or recycled. For example, FIG. 7 shows an embodiment of the invention wherein a second module **102** contains both hydrous zirconium oxide and zirconium phosphate. In certain embodiments, the second module **102** can be reusable as defined herein. The spent dialysate can enter the first module **101.** The spent dialysate can first pass through an activated carbon layer **104.** The spent dialysate can next pass through a first layer of zirconium phosphate **107,** which removes the potassium, calcium and magnesium from the dialysate. Next the spent dialysate can move through the alumina/urease layer **105.** In any embodiments of the first module of the invention, any arrangement of the activated carbon, zirconium phosphate, and urease and alumina layer is contemplated. For example, the fluid can first flow through activated carbon, then enter the urease layer, and then the zirconium phosphate. Alternatively, in any embodiment of the invention, fluid can first flow through the zirconium phosphate layer, then activated carbon, and then enter the urease layer and alumina layer. Still further, the fluid can first flow through the urease layer and alumina layer, then the zirconium phosphate, and then the activated carbon. The fluid can then pass through the connector **103,** and into the second module **102.** The second module **102** contains a hydrous zirconium oxide layer **106,** and a second zirconium phosphate layer **108,** which removes the ammonium ions from the fluid. After dialysis, the reusable module **102** containing the hydrous zirconium oxide and zirconium phosphate can be recharged, discarded, or the sorbent material removed and new material added. In any embodiment of the invention, wash lines may be attached to connector **103** disposed on the reusable module **102** and a second connector positioned after the reusable module **102** wherein the second connector can be positioned thereon or as part of a fluid flow path (not shown).

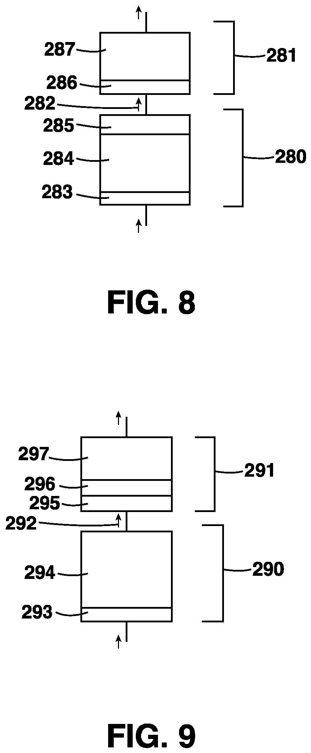

Another non-limiting embodiment of the invention is shown in FIG. 8. Spent dialysate can enter the first, non-reusable, module **280** where it first flows through a layer of activated carbon **283** to remove non-ionic uremic toxins other than urea. The spent dialysate can then enter into a layer of alumina and urease **284,** where the urea is converted to ammonium carbonate and phosphate ions are removed. Next the fluid can enter the hydrous zirconium oxide layer **285,** which removes the remaining phosphate anions and exchanges them with acetate anions. In any embodiment of the first module of the invention, any arrangement of the activated carbon, hydrous zirconium oxide layer, and urease and alumina layer is contemplated. For example, the dialysate can first flow through an activated carbon layer, then the hydrous zirconium oxide layer, and then enter the urease layer and alumina layer. Alternatively, in any embodiment of the invention, the dialysate can first flow through a hydrous zirconium oxide layer, then the activated carbon layer and then enter the urease layer and alumina layer. Still further, the dialysate can first flow through the urease and alumina layer, then the activated carbon layer, and then enter the hydrous zirconium oxide layer. The dialysate can first flow through a hydrous zirconium oxide layer, then through the alumina and urease layers, and then flow through the activated carbon layer. Alternatively, in any embodiment of the invention, the dialysate can first flow through the alumina and urease layer, then through the hydrous zirconium oxide layer, and then through the activated carbon layer. The fluid can then flow through the connector **282,** and into the second, reusable, sorbent module **281.** The sorbent module **281** contains an ion exchange resin layer **286,** and a zirconium phosphate layer **287,** which removes the ammonium ions from the fluid. In any embodiment of the second module of the invention, the fluid can first pass through the zirconium phosphate layer and then the ion exchange resin. Alternatively, in any embodiment of the invention, the sorbent materials in each module may be intermixed as opposed to being arranged in layers. After dialysis, the reusable module **281** containing the zirconium phosphate **287** and ion exchange resin **286** can be recharged, discarded, or the sorbent material removed and new material added. Alternatively, in any embodiment of the invention, the sorbent materials in each module may be intermixed as opposed to being arranged in layers.

In any embodiment of the invention, the hydrous zirconium oxide may be included in a second module as shown in FIG. 9. Spent dialysate can enter a first, non-reusable module **290** where it first flows through a layer of activated carbon **293** to remove non-ionic uremic toxins. The spent dialysate can then enter into a layer of alumina and urease **294,** where the urea is converted to ammonium carbonate and phosphate ions are removed. In any embodiment of the first, non-reusable module **290** of the invention, the dialysate can first flow through the alumina and urease, and then flow through the activated carbon. The fluid can then pass through connector **292** and into the second, reusable module **291.** The second module **291** contains a layer of hydrous zirconium oxide **295,** a layer of ion exchange resin **296** and a layer of zirconium phosphate **297.** In any embodiment of the second module **291** of the invention, any arrangement of the hydrous zirconium oxide, ion exchange resin and zirconium phosphate is contemplated. For example, the fluid may first pass through a layer of ion exchange resin, then pass through a layer of hydrous zirconium oxide and then pass through the zirconium phosphate. Alternatively, in any embodiment of the invention, the fluid may first pass through the ion exchange resin, then pass through the zirconium phosphate and then through the hydrous zirconium oxide. Still further, the fluid may pass through the zirconium phosphate, then through the hydrous zirconium oxide, and then through the ion exchange resin. In any embodiment of the invention, the fluid can first pass through the hydrous zirconium oxide layer, then through the zirconium phosphate layer, and then through the ion exchange resin. Alternatively, in any embodiment of the invention, the fluid can first pass through the zirconium phosphate layer, then the ion exchange resin, and then through the hydrous zirconium oxide layer. The sorbent materials in each module can also be intermixed as opposed to being arranged in layers.

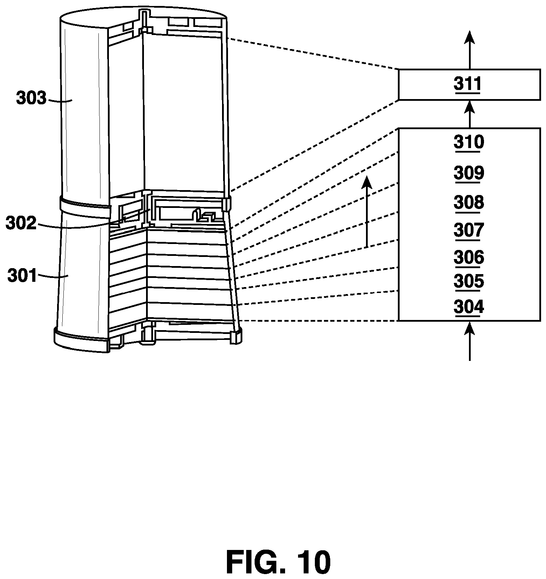

Another non-limiting embodiment of the invention is shown in FIG. 10. A layer of sodium chloride and sodium bicarbonate **304** are disposed on the first module **301.** The sodium chloride and sodium bicarbonate will be dissolved as liquid enters the firs t module **301.** Spent dialysate can enter a first module **301.** In any embodiment of the invention, the first module **301** can be reusable as defined herein. The spent dialysate can then enter a layer of activated carbon **305** to remove non-ionic uremic toxins. The spent dialysate can then enter into a layer of ion-exchange resin **306.** In any embodiment of the invention, this can be a chelating ion exchange resin to selectively remove calcium and magnesium. The dialysate can then enter a layer of alumina and urease **307,** where the urea is converted to ammonium carbonate and phosphate ions are removed. In any embodiment of the invention the urease can be in the form of urease active jack bean meal (JBM). The spent dialysate can next enter a layer of alumina **308.** The fluid can then pass through a layer of hydrous zirconium oxide **309.** In any embodiment of the invention, the hydrous zirconium oxide can be mixed with glass beads. A layer of sodium chloride **310** can be disposed on the end of first module **301,** which will be dissolved by the fluid as it passes through the first module **301.** The fluid then passes out of the first module **301,** through the connector **302,** and into the second module **303.** In any embodiment of the first module **301** of the invention, any arrangement of activated carbon, alumina, urease, ion exchange resin and hydrous zirconium oxide can be used. For example, the fluid can first pass through a layer of sodium chloride and sodium bicarbonate, then activated carbon, then hydrous zirconium oxide, then ion-exchange resin, then alumina and urease and then the sodium chloride. Alternatively, in any embodiment of the invention, the fluid can first pass through a layer of sodium chloride, then ion-exchange resin, then activated carbon, then hydrous zirconium oxide, then alumina and urease, and then sodium chloride. The second module can contain zirconium phosphate **311,** to remove the ammonium ions from solution. In any embodiment of the invention, the zirconium phosphate **311** can be mixed with glass beads.

One skilled in the art will realize that any embodiment of the invention can be included that involve the sorbent materials being mixed within the module, as opposed to arranging the materials in layers. Such mixing of the sorbent materials can be performed interspersing the sorbent materials in a single layer by any method known to those of skill in the art. The arrangements include not just layers of sorbent materials, but also intermixed sorbent materials.

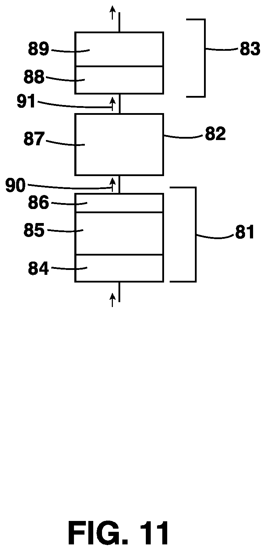

The modular sorbent cartridges of the invention are not limited to having two modules. Any number of modules may be utilized in the invention. A three module sorbent cartridge is shown in FIG 11. The first module **81** contains a layer of activated carbon **84,** a layer of alumina/urease **85,** and a layer of hydrous zirconium oxide **86.** In any embodiment of the invention, any one of the first module **81** second module **82** or third module **83** can be reusable as defined herein. The described layers can also be mixed together rather than being provided in layers. In any embodiment of the invention of the first module of a three module sorbent cartridge, any arrangement of the activated carbon, hydrous zirconium oxide layer, and urease and alumina layer is contemplated. For example, the dialysate can first flow through activated carbon, then the hydrous zirconium oxide layer, and then enter the urease layer and alumina layer. Alternatively, in any embodiment of the invention, the dialysate can first flow through the hydrous zirconium oxide layer, then the activated carbon, then enter the urease layer and alumina layer. Still further, the dialysate can first flow through the urease layer and alumina layer, then the hydrous zirconium oxide layer, and then the activated carbon. Again, the described arrangements include not just layers, but also intermixed sorbent materials. The fluid, after passing through these layers, can pass through a first connector **90,** and into the second module **82.** This second module **82** contains zirconium phosphate **87.** The fluid can then pass through a second connector **91,** and enter a third module **83.** This third module **83** contains a second layer of zirconium phosphate **88,** and a second layer of activated carbon **89** for final purification before passing out of the sorbent cartridge. In any embodiment of the invention of the third module **83** of a three module sorbent cartridge, any arrangement of the activated carbon and the second layer of zirconium phosphate are contemplated. For example, the dialysate can first flow through activated carbon and then the second layer of zirconium phosphate. It will be understood that any number of modules can be configured in the present invention. For example, a sorbent cartridge having four, five, six, seven, or more modules is contemplated by the invention. It will be understood that the described arrangements include not just layers, but also the sorbent materials being intermixed.

As each layer of sorbent material within the modular sorbent cartridge may be recharged, a cartridge is possible where all of the modules are reusable. It is still advantageous to utilize separate modules for the sorbent materials in order to direct the correct recharging solution through the correct module, and because different sorbent materials may need to be replaced more often than others.

Because the ability for the zirconium phosphate layer to bind ammonium ions is finite, while the capacity of the urease layer to break down urea into ammonia is not, it is possible that the capacity of the zirconium phosphate layer may be exceeded. In such a case, excess ammonium ions will be caused to pass through the sorbent cartridge and remain in the dialysate. To protect patient safety, once ammonia breakthrough has occurred, either the dialysis session can be stopped or at least urease can be prevented from catalyzing the conversion of urea to ammonia.

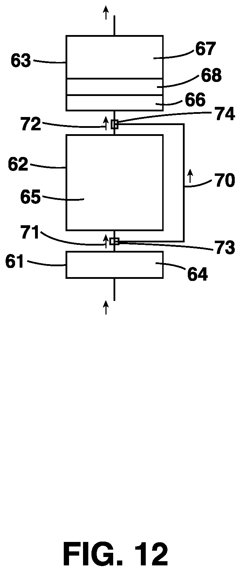

FIG. 12 shows a three-module sorbent cartridge that can allow bypass of the alumina/urease layer in the case of ammonia breakthrough. Ammonia breakthrough can occur when the capacity of the zirconium phosphate layer to exchange ammonium ion is exceeded. In the event of ammonia breakthrough, the spent dialysate can enter the first module **61,** which contains the activated carbon layer **64.** In any embodiment of the invention, any one of the first module **61,** second module **62,** or third module **63** can be reusable as defined herein. The spent dialysate then passes through a first connector **71,** and bypass flow valve **73.** In normal operation, the flow valve **73** can be set to allow the fluid to pass into the second module **62.** The second module **62** contains alumina/urease layer **65,** which catalyzes the breakdown of urea into ammonium ions. In any embodiment of the invention, a single valve can be used and either first valve **73** or second valve **74** can be optional. For example, valve **74** can be optional wherein those skilled in the art will recognize that to alternately direct flow through second module **62** or bypass **70** can be accomplished by a configuring valve **73** as a 3-way valve. Other configurations to achieve the desired alternating direct flow are contemplated by the invention. The fluid then passes through the second connector **72,** by the second valve **74,** and into the third module **63.** The third module **63** can contain a hydrous zirconium oxide layer **66,** ion-exchange resin **68,** and zirconium phosphate layer **67.** In any embodiment of the invention of the third module **63** of a three module sorbent cartridge, any arrangement of the ion-exchange resin, hydrous zirconium oxide layer, and zirconium phosphate layer is contemplated. For example, the dialysate can first flow through ion-exchange resin, then the hydrous zirconium oxide layer, and then enter the zirconium phosphate layer. Alternatively, in any embodiment of the invention, the dialysate can first flow through the hydrous zirconium oxide layer, then the ion-exchange resin, then enter the zirconium phosphate layer. Still further, the dialysate can first flow through the zirconium phosphate layer, then the hydrous zirconium oxide layer, and then the ion-exchange resin. Again, the described arrangements include not just layers, but also intermixed sorbent materials. After passing through the third module, the regenerated dialysate can exit the sorbent cartridge. In the event of ammonia breakthrough, the first valve **73** can be set to redirect the fluid into bypass line **70.** This line will cause the fluid not to enter the second module **62,** and therefore the urea will not be broken down into ammonia in the alumina/urease layer. The fluid will instead be directed to the second valve **74,** which can be optional in any embodiment of the invention, where the fluid enters the second connector **72,** and then the third module **63.** In this way dialysis may continue, while avoiding the creation of ammonia. In any embodiment of the invention either the first valve **73** or the second valve **74** may be optional, and those of skill in the art will recognize that the function can be accomplished with only a single valve if either the first valve **73** or the second valve **74** is a 3-way valve. The valve or valve assembly may also include an access point for a sensor (not shown). The access point can be a portion of the valve assembly wherein a sensor can contact the fluid to take measurement data such as a flow or pressure reading. The form and construction of such access points contemplated by the present invention are those known to one of ordinary skill in the art. In any embodiment of the invention, second valve **74** may be removed and the cartridge may still accomplish the bypass function.

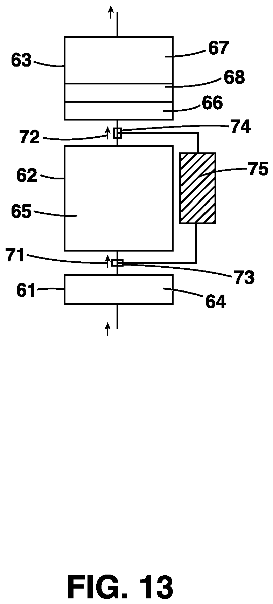

FIG. 13 shows an alternative embodiment of the invention to the sorbent cartridge shown in FIG. 12 wherein a first connector **71** and a flow valve **73** bypass flow through the second module **62** to a component **75.** The component **75** can be a recharger used to recharge or clean the second module **62** while attached to the sorbent cartridge. In any embodiment of the invention, the component **75** can be a container storing a fluid such as a wash fluid or recharging fluid. In any embodiment of the invention, the component **75** can be a pump for pumping fluid. Upon passing through the component **75,** fluid can return through the second connector **72** via the second valve **74,** which can be optional in any embodiment of the invention, and into the third module **63.** In any embodiment of the invention, the component **75** can be removed after a period of time and fluid allowed to flow from the third module **63** through the second connector **72** and the second valve **74,** which can be optional in any embodiment of the invention. The component **75** can be reversibly attached and detached as necessary. In any embodiment of the invention either the first valve **73** or the second valve **74** may be optional, and those of skill in the art will recognize that a desired flow direction and function can be accomplished with only a single valve if either the first valve **73** or the second valve **74** is a 3-way valve. To recharge the sorbent modules in-line, the modules may be connected by wash lines to rechargers, which contain solutions for recharging the modules. As different sorbent materials will be recharging with different solutions, it is beneficial to have the fluid bypass one or more of the modules, and to pass through one or more of the modules.

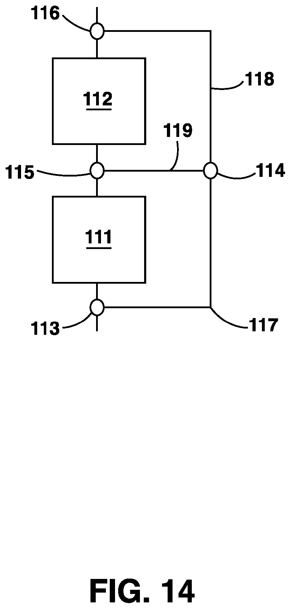

FIG. 14 shows a configuration of the invention that may be utilized to allow fluid to selectively flow or not flow through each of the modules. Before entering the first module **111,** the fluid can pass first valve **113.** In any embodiment of the invention, the first module **111** can be reusable as defined herein. The first valve **113** can either direct fluid into the first module **111,** or into a first bypass line **117.** If the fluid enters the bypass line **117,** the fluid then passes to the second valve **114.** The second valve **114** can direct fluid either through the second bypass line **118,** or through the third bypass line **119** to the third valve **115,** which is in between the two modules. In any embodiment of the invention, valve **114** can be optional wherein the bypass lines can function to move fluid around the modules. In any embodiment of the invention, valve **115** can be optional wherein the bypass lines can function to move fluid around the modules. Here, the fluid would be directed into the second module **112.** Alternatively in any embodiment of the invention, if the fluid was directed through bypass line **118,** then the fluid would go to fourth valve **116** and exit the portion of the system shown. Fluid that passes through the first module **111** also reaches third valve **115.** Here, the third valve **115,** which can be optional, may be set to direct fluid into the second module **112,** or alternatively, in any embodiment of the invention, through the bypass line **119,** which would bypass the second module **112.** In this way, the valves can be set so that fluid is directed through both modules, either one of the modules, or none of the modules. Alternative embodiments of the invention with different valve arrangements are contemplated, for example those of skill in the art will recognize that the same bypass functionality can be accomplished with two valves if valves **116** and **113** are 3-way valves without valves **114** and **115** present.

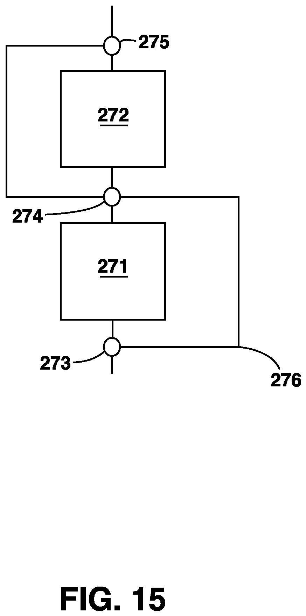

An embodiment of the invention employing a single bypass line is shown in FIG. 15. Before entering the first module **271,** fluid can pass through a first valve **273.** In any embodiment of the invention, the first module **271** can be reusable as defined herein. In any embodiment of invention, the second module **272** can be reusable. First valve **273** can either direct fluid into the first module **271,** or into a bypass line **276.** If the fluid enters the bypass line **276,** the fluid then passes to the second valve **274,** bypassing the first module **271.** Second valve **274** can direct fluid either through the bypass line **276,** or into the second module **272.** If the second valve **274** directs the fluid into the bypass line **276,** the fluid passes to third valve **275,** bypassing the second module **272.** In any embodiment of the invention, alternatives with different valve arrangements are contemplated, for example those of skill in the art will recognize that the same bypass functionality can be accomplished if valves **273** and **275** are 3-way valves without valve **274** present.

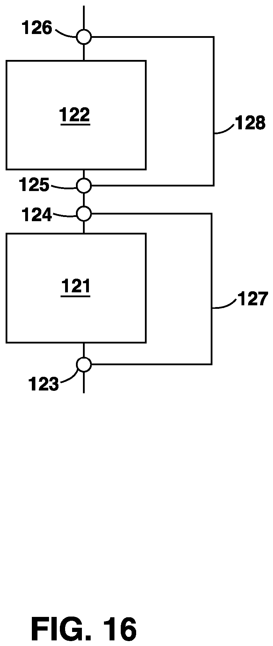

An embodiment of the invention, utilizing separate bypass lines for each module, is shown in FIG. 16. Before the fluid enters the first module **121,** the fluid can pass through the first valve **123.** In any embodiment of the invention, the first module **121** can be reusable as defined herein. The first valve **123** can be set to either direct fluid into the first module **121,** or redirect the fluid into a first bypass line **127** to second valve **124,** which can be optional After passing through the first module **121** or bypassing the first module **121,** the fluid next passes through the third valve **125,** which can be optional. This third valve **125** can be set to either direct the fluid into the second module **122,** or into a second bypass line **128** to the fourth valve **126.** In this way the fluid can be made to flow through both modules, either module or none of the modules. Alternative embodiments of the invention with different valve arrangements are contemplated, for example those of skill in the art will recognize that the same bypass functionality can be accomplished if valves **126** and **123** are 3-way valves without valves **124** and **125** present.

In any embodiment of the invention, the bypass lines can be formed or molded as part of the sorbent body casing. In this manner, the sorbent cartridge has a unitary body with the bypass lines being disposed thereon. One or more of the modules can be detachable from the unitary sorbent body cartridge having the bypass lines molded thereon. This design avoids the need for tubing or separate lines. In any embodiment of the invention, the connectors can form the bypass lines.

As demonstrated in FIG.'s. 14, 15, and 16, the precise number of valves or connectors utilized in any embodiment of the invention may be altered without being beyond the scope of the invention. Valves and connectors may be added or removed to any of the embodiments of the invention shown to accomplish the same end. Further, the functions of a four-way valve may be accomplished with two three-way valves or three two-way valves.

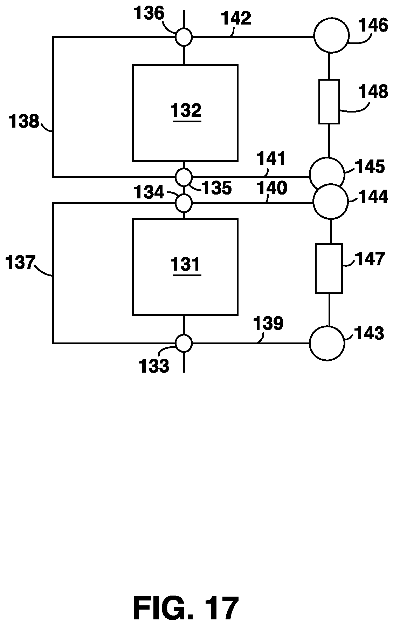

The sorbent module may be connected to multiple rechargers and wash lines, so that multiple layers of sorbent material may be recharged without having to clean out the rechargers and lines and add new wash fluid. FIG. 17 shows an embodiment of the invention utilizing multiple wash lines, bypass lines and rechargers. The first recharger **147** can be connected to recharger nodes **143** and **144.** A first wash line **139** can connect the first recharger node **143** to a first valve **133,** which is positioned before the first module **131.** In any embodiment of the first, second or third aspects of the invention, the first module **131** can be reusable as defined herein. The first valve **133** can be set to direct the fluid into the first module **131,** or alternatively to bypass the first module **131** through bypass line **137** which connects to a second valve **134** after the first module **131,** or as a third alternative to direct fluid through the first wash line **139** to circulate between the first module **131** and the first recharger **147.** The second recharger node **144** can be connected to this second valve **134** by a second wash line **140.** Fluid flowing from the first bypass line **137** or from the first module **131** can flow through the second wash line **140** to the second recharger node **144** and then into the first recharger **147,** or can pass to third valve **135,** which can either direct the fluid into the second module **132,** or alternatively to bypass the second module **132** through the second bypass line **138,** which connects to the fourth valve **136** positioned after the second module. A third recharger node **145** can also be connected to the third valve **135** by third wash line **141,** and can connect to a second recharger **148.** The third valve **135** can direct fluid through the third wash line **141** to circulate between the second module **132** and second recharger **148,** or alternatively through the second bypass line **138,** or in the alternative to the second module **132.** A fourth recharger node **146** can be attached by a fourth wash line **142** to the fourth valve **136.** Fluid from the second module **132** or from the second bypass line **138** can selectively be directed through the fourth valve **136** into the fourth wash line **142** to the second recharger **148.** Alternative embodiments of the invention with different valve arrangements are contemplated, for example those of skill in the art will recognize that the same bypass functionality can be accomplished for valve **133** represented by a configuration of 2-way valves separately on each of first bypass line **137,** first wash line **139** and a connection to first module **131.** Similarly, the same bypass functionality can be accomplished for valve **135** represented by a configuration of 2-way valves separately on each of second bypass line **138,** second wash line **139** and a connection to second module **131** without valve **134** present. In any embodiment of the invention, bypass lines **137** and **138,** and wash lines **139** and **141** can be directly connected to the connectors between the modules, rather than to valves **134** and **136,** and **133** and **135,** respectively.

By utilizing the rechargers and bypass lines, different recharger fluids may be passed through the appropriate modules. The valves may be set open to the wash lines and closed to the connector, so that fluid is directed to the recharger. Alternatively, the first valve **133** may be set open to the wash line **139** and connector, while the second valve **134** is closed to the second module **132,** so that fluid is made to circulate between the first module **131** and the recharger **147.** If the first valve **133** is open to the bypass line **137,** but closed to the connector, then fluid will be directed through the bypass line **137** around the first module **131.** Finally, if the first valve **133** is open to the connector and the second valve **134** and third valve **135** are open to the second connector, the fluid will be directed through both modules.

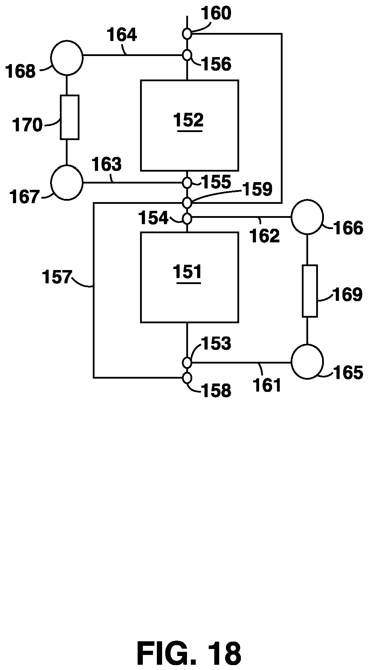

Another embodiment of the invention, utilizing a single bypass line, is shown in FIG. 18. The first recharger node **165** can be connected to a first valve **153,** positioned before the first module **151,** by a first wash line **161.** In any embodiment of the invention, the first module **151** can be reusable as defined herein. In any embodiment of the invention, the second module **152** can be reusable. A second recharger node **166** can be connected to a second valve **154,** positioned after the first module **151,** by a second wash line **162.** The first recharger node **165** and second recharger node **166** can connect the first recharger **169.** A third recharger node **167** can be connected to a third valve **155,** positioned before the second module **152,** by a third wash line **163.** A fourth recharger node **168** can be connected to a fourth valve **156,** positioned after the second module **152,** by a fourth wash line **164.** The third recharger node **167** and fourth recharger node **168** can connect the second recharger **170.** A fifth valve **158,** can be positioned before the first module **151,** and connect to a bypass line **157,** which directs fluid around the first module **151** to a sixth valve **159,** positioned between the first and second modules, **151** and **152,** respectively. The bypass line **157** can be further connected to a seventh valve **160,** positioned after the second module **152.** Other arrangements of valves are contemplated by the invention and those of skill in the art will recognize other combinations of valves that can be employed to accomplish these flow paths. For example, in any embodiment of the invention, first valve **153,** fourth valve **156** and sixth valve **159** can be optional and the flow path functions can be accomplished with four valves if fifth valve **158,** second valve **154,** third valve **155** and seventh valve **160** are three-way valves. The valves can be set to allow fluid to flow into the modules, through the bypass lines, or into the rechargers in several combinations.

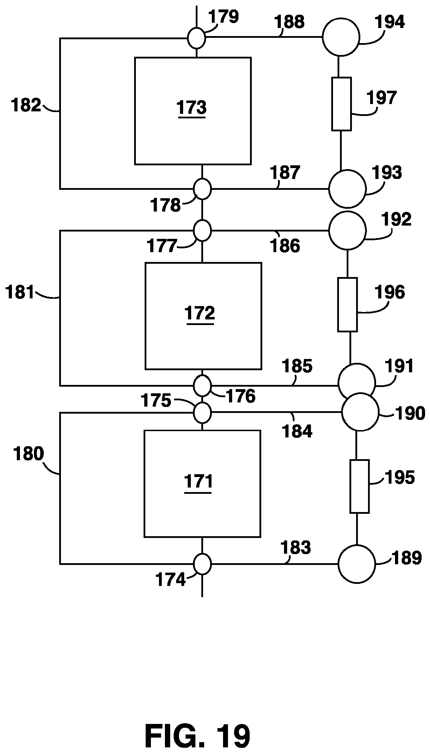

One skilled in the art will recognize that this system is not limited to modular sorbent cartridges having two modules. Additional modules may be utilized with additional bypass lines and rechargers. FIG. 19 shows a three-module system for in-line module recharging. A first valve **174** can be connected to a first module **171,** a first bypass line **180,** and a first wash line **183.** In any embodiment of the invention, the first module **171** can be reusable as defined herein. The first wash line **183** is connected to a first recharger node **189.** The first valve **174** may direct fluid into the first module **171** or into the first bypass line **180,** which connects to a second valve **175** positioned after the first module **171.** The second valve **175** can also be connected to a second wash line **184,** and then to a second recharger node **190.** The first recharger node **189** and second recharger node **190** can connect the first recharger **195.** The fluid then travels to the third valve **176,** positioned before the second module **172.** The third valve **176** can be attached to a third wash line **185,** which connects a third recharger node **191.** This third valve **176** can direct fluid into the second module **172,** or into a second bypass line **181,** which connects to a fourth valve **177,** positioned after the second module **172.** This fourth valve **177** can also connect to a fourth wash line **186,** which is connected to a fourth recharger node **192.** The third recharger node **191** and fourth recharger node **192** can connect the second recharger **196.** The fluid can then pass to a fifth valve **178,** positioned before the third module **173.** The fifth valve **178** can also be attached to a fifth wash line **187**, which connects to a fifth recharger node **193**. The fifth valve can direct the fluid into the third module **173**, or into a third bypass line **182**, which connects to a sixth valve **179**, positioned after the third module **173**. The sixth valve **179** can also be attached to a sixth wash line **188**, which connects to a sixth recharger node **194**. The fifth recharger node **193** and sixth recharger node **194** can connect the third recharger **197**. The valves can be set to allow fluid to flow into the modules, through the bypass lines, or into the rechargers in several combinations. Further, additional valve arrangement known to those of ordinary skill are contemplated. In any embodiment of the invention, bypass lines **180**, **181** and **182**, and wash lines **183**, **185** and **187** can be directly connected to the connectors between the modules, rather than to valves **175**, **177** and **179**, and valves **174**, **176** and **178**, respectively.

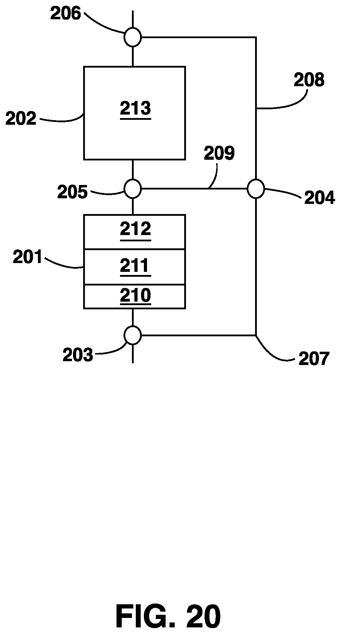

Various sorbent materials may be used in each of the modules of the sorbent cartridge. A non-limiting example is shown in FIG. 20. In any embodiment of the invention, the first module **201** can be reusable as defined herein. In any embodiment of the invention, the second module **202** can be reusable. The first module **201** can contain layers of activated carbon **210,** alumina/urease **211,** and hydrous zirconium oxide **212.** The second module **202** can contain zirconium phosphate **213.** After dialysis, the layers may be recharged in each module. Fluid capable of recharging activated carbon, hydrous zirconium oxide, and alumina/urease may be passed through the first valve **203**, and into the first module **201.** The fluid can then pass out of the first module **201** to second valve **205**. This fluid may then be redirected around the second module **202** into bypass line **209**. The fluid can pass to third valve **204,** into third bypass line **208**, and to the fourth valve **206**, thus bypassing the second module **202**. Alternatively, fluid for recharging zirconium phosphate **213** may be introduced. The fluid will pass to valve **203**, and be redirected into bypass line **207** to third valve **204**. The fluid may then be redirected through bypass line **209** to the second valve **205**. Here the fluid will be directed to flow into the second module **202**. This allows recharging and recycling of either module without introducing the recharging fluid into the other module. The sorbent materials within each module may be in any order. Alternatively, the sorbent materials within a module may be intermixed. Further, additional valve arrangements known to those of ordinary skill are contemplated.

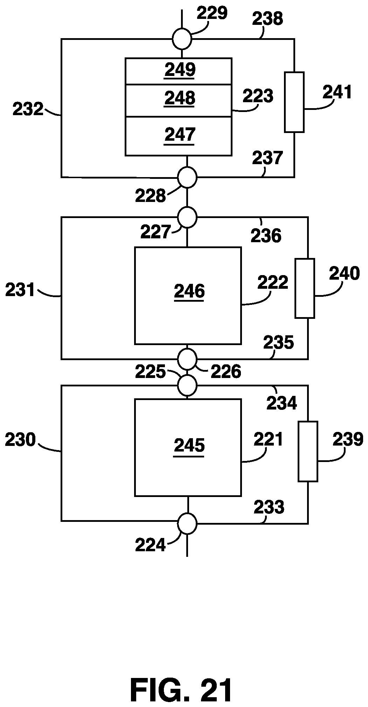

Another embodiment of the invention is the 3-module cartridge shown in FIG. 21. A first valve **224** can be connected to a first module **221,** a first bypass line **230**, and a first wash line **233.** The wash line is connected to a first recharger **239.** The first valve **224** may direct fluid into the first module **221** or into the first bypass line **230**, which can connect to a second valve **225** positioned after the first module **221.** The second valve **225** can also connect to a second wash line **234,** and then to the first recharger **239.** The fluid can then travel to the third valve **226,** positioned before the second module **222.** Third valve **226** can attach to a third wash line **235,** which connects a second recharger **240**. This third valve **226** can direct fluid into the second module **222,** or into a second bypass line **231,** which can connect to a fourth valve **227,** positioned after the second module **222.** The fourth valve **227** can also connect to a fourth wash line **236,** which is connected to the second recharger **240**. The fluid can then pass to a fifth valve **228,** positioned before the third module **223.** The fifth valve **228** can also attach to a fifth wash line **237,** which connects to a third recharger **241.** The fifth valve **228** can direct the fluid into the third module **223,** or into a third bypass line **232,** which connects to a sixth valve **229,** positioned after the third module **223.** The sixth valve **229** can also attach to a sixth wash line **238,** which connects to the third recharger **241.** The first module **221** can contain activated carbon **245.** The second module **222** can contain alumina/urease **246.** In any embodiment of the invention, any one of the first module **221,** second module **222,** or third module **223** can be reusable as defined herein. The third module **223** can contain hydrous zirconium oxide **247**, ion exchange resin **248**, and zirconium phosphate **249**. Further, additional valve arrangement known to those of ordinary skill are contemplated. In any embodiment of the third module of a three module sorbent cartridge of the invention, any arrangement of the ion-exchange resin, hydrous zirconium oxide layer, and zirconium phosphate layer is contemplated. For example, the dialysate can first flow through ion-exchange resin, then the hydrous zirconium oxide layer, and then enter the zirconium phosphate layer. Alternatively, in any embodiment of the invention, the dialysate can first flow through the hydrous zirconium oxide layer, then the ion-exchange resin, then enter the zirconium phosphate layer. Still further, in any embodiment of the invention, the dialysate can first flow through the zirconium phosphate layer, then the hydrous zirconium oxide layer, and then the ion-exchange resin. Again, in any embodiment of the invention the described arrangements include not just layers, but also intermixed sorbent materials. After use, any of the spent modules may be discarded and replaced. They may also be recharged. The activated carbon **245** may be recharged by passing through the first module **221** a fluid of heated water. The alumina/urease **246** may be recharged by first passing a fluid of heated water through second module **222**, and then passing a solution containing urease through. The third module **223**, containing hydrous zirconium oxide **247**, ion exchange resin **248**, and zirconium phosphate **249** may be recharged by passing through the module a solution containing hydrogen and sodium ions. By selectively directing fluid through modules, or through the bypass lines, each of the modules may be recharged for further use. In any embodiment of the invention, bypass lines **230**, **231** and **232,** and wash lines **233, 235** and **237** can be directly connected to the connectors between the modules, rather than to valves **225, 227** and **229,** and **224, 226** and **228,** respectively.

One skilled in the art will recognize that the precise order of sorbent materials within the modules, or the module in which a particular sorbent material is contained, may be modified without detracting from the invention. In any embodiment of the invention the sorbent materials can be arranged differently within the sorbent modules. Further, in any embodiment of the invention, the sorbent materials may be mixed within the modules, as opposed to arranging the material in layers.

In any embodiment of the invention, one or more of the modules may also be made detachable. This will enable the detachment of one or more modules without detaching the others. The detached modules can be discarded and replaced, recharged, or the sorbent material may be discarded, the module refilled, and then reused. To protect against cross-usage by patients, the detachable modules may be furnished with an identification component, such as a barcode. This will enable the same detachable module to be matched to a particular patient, and thereby avoid use of the module by another patient.

In order to ensure that all of the residual fluid is removed from the reusable modules, valves, bypass lines and wash lines, it may be advantageous to blow a gas, such as air, or an inert gas such as argon, through the module. In any embodiment of the invention, the gas may be air, filtered air, nitrogen, helium, or other gas. The wash line may be adapted so that a gas may be blown through the module instead of, or in addition to, a wash liquid.

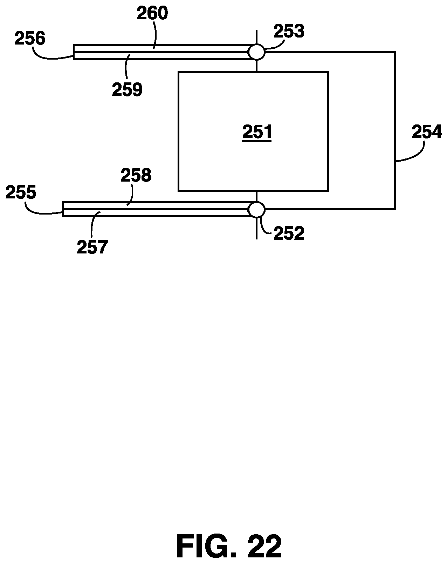

In any embodiment of the invention, a wash line may be divided into two lines as shown in FIG. 22. FIG. 22 shows a first module **251** from an embodiment of the invention similar to that shown in FIG. 17, wherein the wash line is adapted to utilize both a liquid and a gas. In any embodiment of the invention, the first module **251** can be reusable as defined herein. The first wash line **255** can connect to a first valve **252** positioned before the first module **251**. The first valve **252** can also connect to the first bypass line **254**. The first bypass line **254** can direct either liquid or gas around the first module **251** to the second valve **253**. The first wash line **255** can be further divided into two lines. These lines are the liquid wash line **257,** and the gas wash line **258**. A second wash line **256** can connect to the second valve **253**, and also have both a liquid wash line **259** and a gas wash line **260**. This embodiment of the invention allows both gas and liquid to pass either into the first module, or into the bypass line and around the first module. A second module, second bypass line, and third and fourth fluid lines are attached, so that fluid or gas may selectively be made to enter either module, or to bypass them.

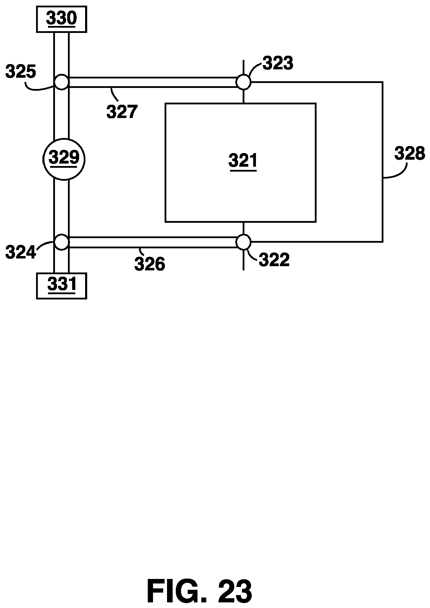

FIG. 23 shows an alternative embodiment of the invention utilizing both a gas and liquid line. The first wash line **326** can connect a first valve **324** to a second valve **322**. The first valve **324** can be connected to the recharger connector **329**, and a fluid collector **331**. The recharger connector **329** can also be attached to third valve **325**. Third valve **325** can connect the gas source **330** and fourth valve **323** via second wash line **327**. Both the second valve **322** and the fourth valve **323** connect to the module **321** and a bypass line **328**. This embodiment of the invention allows both gas and liquid to circulate through the module **321**, or around the module.

In addition to dividing the wash line into a gas wash line and a liquid wash line, the wash line may be divided into two different liquid lines. This enables different liquids to travel between the recharger and the modules.

To make use of the modular sorbent cartridge easier, the valve or valve assembly may be operated by a programmable controller or computer system that can be programmed to regulate flow through the valves and into and out of the modules. An optical sensor, photocell or other flow sensing apparatus may detect the flow of fluid through any two points in the sorbent cartridge. For example, an optical fluid flow device can be provided for measuring flow wherein the device includes an optical fluid pressure measuring device having sensors positioned in any one of the flow paths between the modules, in the connectors, or in the valve assemblies. Preferably, the sensors will be placed in a passageway defined between the modules. In any embodiment of the invention, the optical fluid sensors can be connected to an interferometer associated with an opto-electronic demodulator which has an output signal representing the differential pressure between the two sensed areas. In any embodiment of the invention, a flow sensing apparatus can have a flow-responsive element projecting into a fluid flow path, and a position sensor associated with the element which detects a change in position of the flow responsive element in response to the fluid flow. The flow-responsive element can be made of a wide variety of materials having the desired properties known to those of ordinary skill in the art.

One skilled in the art will understand that various combinations and/or modifications and variations can be made in the dialysis system depending upon the specific needs for operation.

## Claims

1. A sorbent cartridge, comprising:
at least one reusable module (131) containing one or more rechargeable sorbent materials, and a second module (132) connected in series and forming a sorbent cartridge, the at least one reusable module having one or more connectors (133,134,135,136) selectively fluidly connectable to any part of a fluid flow path selected from any one of a wash line (183), a bypass line (180), a fluid line or the second module;
wherein the wash line (183) is a line fluidly connectable to a recharger (148) to direct fluid between the recharger and the reusable module;
wherein the bypass line (180) is a line fluidly connectable to another module; and
wherein the fluid line is fluidly connectable to any portion of a dialysis circuit; and
wherein the recharger contains solutions for recharging the sorbent material within the reusable module; said sorbent cartridge further comprising at least one valve (174,175,176,177) positioned before and/or after the modules on the connectors to selectively direct flow through at least one module, wash line, recharger, or bypass line; wherein the reusable module is configured to be in an inline state by being fluidly connected to any one of the fluid line or the bypass line, and wherein the reusable module is configured to be in an off-line state by being fluidly connected to the recharger,
wherein "in-line" refers to a state in which the module is fluidly connected to a dialysis machine, dialysis flow path or dialysis circuit, and wherein "off-line" refers to a state in which the module is fluidly disconnected from a dialysis machine, dialysis flow path or dialysis circuit.

2. The sorbent cartridge of claim 1, wherein: a first bypass line (180) connects a first valve positioned on a connector before the reusable module to a second valve (175); a second bypass line (181) connects the second valve to a third valve (176) positioned on a connector between the reusable module and second module; and a third bypass line (182) connects the second valve to a fourth valve (177) positioned on a connector after the second module; or
wherein a bypass line (180) connects a first valve (174) positioned on a connector before the reusable module to a second valve (175) positioned on a connector between the first and second module, and connects the second valve to a third valve (176) positioned on a connector after the second module, the sorbent cartridge further comprising any one of a four-way, three-way, two-way valve, or combinations thereof, positioned before and/or after the modules on the connectors to selectively direct fluid flow through the modules , wash line, recharger and/or bypass line; or
wherein a first bypass line connects a first valve (174) positioned on a connector before the reusable module to a second valve (175) positioned on a connector between the reusable module and the second module; and a second bypass line (181) connects a third valve (176) positioned on the connector between the reusable module and second module to a fourth valve (177) positioned on a connector after the second module; or
wherein a first bypass line (180) connects a first valve (174) positioned on a connector before the reusable module to a second valve (175) positioned on a connector between the first and second modules; a second bypass line (181) connects a third valve (176) positioned on the connector between the reusable module and second module to a fourth valve (177) positioned on a connector after the second module; a first wash line (183) connects the first valve to a first recharger connector; a second wash line (184) connects the second valve to a second recharger connector; a third wash line (185) connects the third valve to a third recharger connector; and a fourth wash line (186) connects the fourth valve to a fourth recharger connector; or
wherein a first valve (174) positioned on a connector before the reusable module connects a first wash line (183) to a first recharger connector; a second valve (175) positioned on a connector between the reusable module and second module connects a second wash line (184) to a second recharger connector; a third valve (176) positioned on the connector between the first and second modules connects a third wash line (185) to a third recharger connector; a fourth valve (177) positioned on a connector after the second module connects a fourth wash line (186) to a fourth recharger connector; a fifth valve (178) positioned on the connector before the reusable module connects a bypass line to a sixth valve (179) positioned on the connector between the reusable module and second module, and further connects to a seventh valve positioned on the connector after the second module.

3. The sorbent cartridge of claim 1, or 2, wherein the reusable and second modules are part of a controlled compliant dialysis circuit (380).

4. The sorbent cartridge of any preceding claim , wherein the valves are operated under control of a programmable controller or computer system to regulate flow into, out of, and between modules.

5. The sorbent cartridge of any preceding claim, wherein fluid flow through the valves is sensed by a photocell or other flow sensing and/or measuring apparatus (348).

6. The sorbent cartridge of any preceding claim, further comprising a control pump (375) for circulating fluid in the fluid flow path.

7. A method of recharging a sorbent in a sorbent cartridge, comprising at least one reusable module containing one or more rechargeable sorbent materials, and a second module, the at least one reusable module having one or more connectors selectively fluidly connectable to any part of a fluid flow path selected from any one of a wash line, a bypass line, a fluid line or the second module, wherein the method of recharging the sorbent comprises the steps of:
connecting at least one reusable module and the second module in series with one or more connectors; selectively:
fluidly connecting at least one connector to at least one wash line, wherein the wash line is fluidly connected to a recharger containing solutions for recharging the sorbent material within the reusable module;
fluidly connecting at least one connector to a bypass line wherein the bypass line diverts flow from the connector to bypass at least one module;
connecting one or more valves to the connectors at junctions between the modules, was lines and/or bypass lines; and
selectively opening and closing the valves to selectively direct flow through the connectors, modules, wash lines and/or bypass lines, to set the reusable module to be in an off-line state by being fluidly connectable to the recharger and to set the reusable module to be in an in-line state by being fluidly connectable to any one of the fluid line or the bypass line,
wherein "in-line" refers to a state in which the module is fluidly connected to a dialysis machine, dialysis flow path or dialysis circuit, and wherein "off-line" refers to a state in which the module is fluidly disconnected from a dialysis machine, dialysis flow path or dialysis circuit.

8. The method of claim 7, further comprising a valve positioned on a connector before the reusable module, the valve connecting the connector, a wash line and a bypass line, wherein the valve is open to the wash line, and closed to the connector and bypass line such that flow is directed to a recharger; or
further comprising a valve positioned on a connector before a reusable module, the valve connecting the connector, a wash line and a bypass line, wherein the valve is open to the wash line and connector, and closed to the bypass line such that flow circulates between the reusable module and the recharger, but wherein a second valve positioned on a connector between the reusable module and a second module is closed such that flow cannot continue from the reusable module to the second module; or
further comprising a valve positioned on a connector before the reusable module, the valve connecting the connector, a wash line and a bypass line, wherein the valve is open to the bypass line, and closed to the wash line and the connector, such that flow is directed through the bypass line to bypass the reusable module; or
further comprising a valve positioned on a connector before the reusable module, the valve connecting the connector, a wash line and a bypass line, wherein the valve is open to the connector, and closed to the bypass and wash lines, such that flow is directed through the connector and through the reusable module.

9. The method of claim 7, further comprising a pump attached to the recharger or wash line.

10. The method of claim 7, further comprising an inert gas selected from any one of argon, air, filtered air, nitrogen, and helium to blow out the module.

11. The method of claim 7, wherein the wash lines are subdivided into a top and a bottom wash line, and
wherein the top line is a fluid line and the bottom line is a gas line, or
wherein the top line is a gas line and the bottom line is a fluid line, or
wherein both the top line and the bottom line are fluid lines.

## Patentansprüche

1. Sorbenskartusche, die Folgendes umfasst:
wenigstens ein wiederverwendbares Modul (131), das ein oder mehrere anreicherbare Sorbensmaterialien enthält, und ein zweites Modul (132), das in Reihe verbunden ist und eine Sorbenskartusche ausbildet, wobei das wenigstens eine wiederverwendbare Modul einen oder mehrere Verbinder (133, 134, 135, 136) aufweist, die mit einen beliebigen Teil eines Fluidströmungswegs wahlweise fluidisch verbindbar sind, der aus einer Waschleitung (183), einer Umgehungsleitung (180), einer Fluidleitung oder dem zweiten Modul ausgewählt ist;
wobei die Waschleitung (183) eine Leitung ist, die mit einer Anreicherungsvorrichtung (148) fluidisch verbindbar ist, um Fluid zwischen der Anreicherungsvorrichtung und dem wiederverwendbaren Modul zu leiten;
wobei die Umgehungsleitung (180) eine Leitung ist, die mit einem anderen Modul fluidisch verbindbar ist; und
wobei die Fluidleitung mit einem beliebigen Abschnitt eines Dialysekreislaufs fluidisch verbindbar ist; und
wobei die Anreicherungsvorrichtung Lösungen zum Anreichern des Sorbensmaterials innerhalb des wiederverwendbaren Moduls enthält; wobei die Sorbenskartusche ferner wenigstens ein Ventil (174, 175, 176, 177) umfasst, das vor und/oder nach den Modulen an den Verbindern positioniert ist, um eine Strömung durch wenigstens ein Modul, eine Waschleitung, eine Anreicherungsvorrichtung oder eine Umgehungsleitung wahlweise zu leiten; wobei das wiederverwendbare Modul konfiguriert ist, um dadurch, dass es mit der Fluidleitung oder der Umgehungsleitung fluidisch verbunden ist, in einem Inline-Zustand zu sein, und wobei das wiederverwendbare Modul konfiguriert ist, um dadurch, dass es mit der Anreicherungsvorrichtung fluidisch verbunden ist, in einem Offline-Zustand zu sein,
wobei sich "Inline" auf einen Zustand bezieht, in dem das Modul mit einer Dialysemaschine, einem Dialyseströmungweg oder einem Dialysekreislauf fluidisch verbunden ist, und wobei sich "Offline" auf einen Zustand bezieht, in dem das Modul von einer Dialysemaschine, einem Dialyseströmungweg oder einem Dialysekreislauf fluidisch getrennt ist.

2. Sorbenskartusche nach Anspruch 1, wobei: eine erste Umgehungsleitung (180) ein erstes Ventil, das an einem Verbinder vor dem wiederverwendbaren Modul positioniert ist, mit einem zweiten Ventil (175) verbindet; eine zweite Umgehungsleitung (181) das zweite Ventil mit einem dritten Ventil (176) verbindet, das an einem Verbinder zwischen dem wiederverwendbaren Modul und dem zweiten Modul positioniert ist; und eine dritte Umgehungsleitung (182) das zweite Ventil mit einem vierten Ventil (177) verbindet, das an einem Verbinder nach dem zweiten Modul positioniert ist; oder
wobei eine Umgehungsleitung (180) ein erstes Ventil (174), das an einem Verbinder vor dem wiederverwendbaren Modul positioniert ist, mit einem zweiten Ventil (175) verbindet, das an einem Verbinder zwischen dem ersten und dem zweiten Modul positioniert ist, und das zweite Ventil mit einem dritten Ventil verbindet (176), das an einem Verbinder nach dem zweiten Modul positioniert ist, wobei die Sorbenskartusche ferner ein Vierwege-, ein Dreiwege-, ein Zweiwegeventil oder Kombinationen davon umfasst, die vor und/oder nach den Modulen an den Verbindern positioniert sind, um eine Fluidströmung durch die Module, die Waschleitung, die Anreicherungsvorrichtung und/oder die Umgehungsleitung wahlweise zu leiten; oder
wobei eine erste Umgehungsleitung ein erstes Ventil (174), das an einem Verbinder vor dem wiederverwendbaren Modul positioniert ist, mit einem zweiten Ventil (175) verbindet, das an einem Verbinder zwischen dem wiederverwendbaren Modul und dem zweiten Modul positioniert ist; und eine zweite Umgehungsleitung (181) ein drittes Ventil (176), das an dem Verbinder zwischen dem wiederverwendbaren Modul und dem zweiten Modul positioniert ist, mit einem vierten Ventil (177) verbindet, das an einem Verbinder nach dem zweiten Modul positioniert ist; oder
wobei eine erste Umgehungsleitung (180) ein erstes Ventil (174), das an einem Verbinder vor dem wiederverwendbaren Modul positioniert ist, mit einem zweiten Ventil (175) verbindet, das an einem Verbinder zwischen dem ersten und dem zweiten Modul positioniert ist; eine zweite Umgehungsleitung (181) ein drittes Ventil (176), das an dem Verbinder zwischen dem wiederverwendbaren Modul und dem zweiten Modul positioniert ist, mit einem vierten Ventil (177) verbindet, das an einem Verbinder nach dem zweiten Modul positioniert ist; eine erste Waschleitung (183) das erste Ventil mit einem ersten Anreicherungsverbinder verbindet; eine zweite Waschleitung (184) das zweite Ventil mit einem zweiten Anreicherungsverbinder verbindet; eine dritte Waschleitung (185) das dritte Ventil mit einem dritten Anreicherungsverbinder verbindet; und eine vierte Waschleitung (186) das vierte Ventil mit einem vierten Anreicherungsverbinder verbindet; oder
wobei ein erstes Ventil (174), das an einem Verbinder vor dem wiederverwendbaren Modul positioniert ist, eine erste Waschleitung (183) mit einem ersten Anreicherungsverbinder verbindet; ein zweites Ventil (175), das an einem Verbinder zwischen dem wiederverwendbaren Modul und dem zweiten Modul positioniert ist, eine zweite Waschleitung (184) mit einem zweiten Anreicherungsverbinder verbindet; ein drittes Ventil (176), das an dem Verbinder zwischen dem ersten und dem zweiten Modul positioniert ist, eine dritte Waschleitung (185) mit einem dritten Anreicherungsverbinder verbindet; ein viertes Ventil (177), das an einem Verbinder nach dem zweiten Modul positioniert ist, eine vierte Waschleitung (186) mit einem vierten Anreicherungsverbinder verbindet; ein fünftes Ventil (178), das an dem Verbinder vor dem wiederverwendbaren Modul positioniert ist, eine Umgehungsleitung mit einem sechsten Ventil (179) verbindet, das an dem Verbinder zwischen dem wiederverwendbaren Modul und dem zweiten Modul positioniert ist, und ferner mit einem siebten Ventil verbunden ist, das an dem Verbinder nach dem zweiten Modul positioniert ist.

3. Sorbenskartusche nach Anspruch 1 oder 2, wobei das wiederverwendbare und das zweite Modul Teil eines gesteuerten konformen Dialysekreislaufs (380) sind.

4. Sorbenskartusche nach einem der vorhergehenden Ansprüche, wobei die Ventile unter einer Steuerung einer programmierbaren Steuereinheit oder eines Computersystems betrieben werden, um die Strömung in Module hinein, aus Modulen heraus und zwischen Modulen zu regulieren.

5. Sorbenskartusche nach einem der vorhergehenden Ansprüche, wobei die Fluidströmung durch die Ventile durch eine Fotozelle oder eine andere Strömungserfassungs- und/oder Strömungsmessungseinrichtung (348) erfasst wird.

6. Sorbenskartusche nach einem der vorhergehenden Ansprüche, die ferner eine Steuerpumpe (375) zum Zirkulieren von Fluid in dem Fluidströmungsweg umfasst.

7. Verfahren zum Anreichern eines Sorbens in einer Sorbenskartusche, die wenigstens ein wiederverwendbares Modul, das ein oder mehrere anreicherbare Sorbensmaterialien enthält, und ein zweites Modul umfasst, wobei das wenigstens eine wiederverwendbare Modul einen oder mehrere Verbinder aufweist,
die mit einem beliebigen Teil eines Fluidströmungswegs wahlweise fluidisch verbindbar sind, der aus einer Waschleitung, einer Umgehungsleitung, einer Fluidleitung oder dem zweiten Modul ausgewählt ist, wobei das Verfahren zum Anreichern des Sorbens die folgenden Schritte umfasst:
Verbinden wenigstens eines wiederverwendbaren Moduls und des zweiten Moduls in Reihe mit einem oder mehreren Verbindern; wahlweise:
fluidisches Verbinden wenigstens eines Verbinders mit wenigstens einer Waschleitung, wobei die Waschleitung mit einer Anreicherungsvorrichtung fluidisch verbunden ist, die Lösungen zum Anreichern des Sorbensmaterials innerhalb des wiederverwendbaren Moduls enthält;
fluidisches Verbinden wenigstens eines Verbinders mit einer Umgehungsleitung, wobei die Umgehungsleitung eine Strömung von dem Verbinder umleitet, um wenigstens ein Modul zu umgehen;
Verbinden eines oder mehrerer Ventile mit den Verbindern an Anschlussstellen zwischen den Modulen, den Waschleitungen und/oder den Umgehungsleitungen; und
wahlweises Öffnen und Schließen der Ventile, um die Strömung durch die Verbinder, die Module, die Waschleitungen und/oder die Umgehungsleitungen wahlweise zu leiten, um das wiederverwendbare Modul dadurch, dass es mit der Anreicherungsvorrichtung fluidisch verbindbar ist, in einen Offline-Zustand zu versetzen, und um das wiederverwendbare Modul dadurch, dass es mit der Fluidleitung oder der Umgehungsleitung fluidisch verbindbar ist, in einen Inline-Zustand zu versetzen,
wobei sich "Inline" auf einen Zustand bezieht, in dem das Modul mit einer Dialysemaschine, einem Dialyseströmungweg oder einem Dialysekreislauf fluidisch verbunden ist, und wobei sich "Offline" auf einen Zustand bezieht, in dem das Modul von einer Dialysemaschine, einem Dialyseströmungweg oder einem Dialysekreislauf fluidisch getrennt ist.

8. Verfahren nach Anspruch 7, das ferner ein Ventil umfasst, das an einem Verbinder vor dem wiederverwendbaren Modul positioniert ist, wobei das Ventil den Verbinder, eine Waschleitung und eine Umgehungsleitung verbindet, wobei das Ventil derart für die Waschleitung geöffnet und für den Verbinder und die Umgehungsleitung geschlossen ist, dass die Strömung zu einer Anreicherungsvorrichtung geleitet wird; oder
das ferner ein Ventil umfasst, das an einem Verbinder vor einem wiederverwendbaren Modul positioniert ist, wobei das Ventil den Verbinder, eine Waschleitung und eine Umgehungsleitung verbindet, wobei das Ventil derart für die Waschleitung und den Verbinder geöffnet und für die Umgehungsleitung geschlossen ist, dass die Strömung zwischen dem wiederverwendbaren Modul und der Anreicherungsvorrichtung zirkuliert, wobei jedoch ein zweites Ventil, das an einem Verbinder zwischen dem wiederverwendbaren Modul und einem zweiten Modul positioniert ist, derart geschlossen ist, dass die Strömung von dem wiederverwendbaren Modul nicht zu dem zweiten Modul weiterführen kann; oder
das ferner ein Ventil umfasst, das an einem Verbinder vor dem wiederverwendbaren Modul positioniert ist, wobei das Ventil den Verbinder, eine Waschleitung und eine Umgehungsleitung verbindet, wobei das Ventil derart für die Umgehungsleitung geöffnet und für die Waschleitung und den Verbinder geschlossen ist, dass die Strömung durch die Umgehungsleitung geleitet wird, um das wiederverwendbare Modul zu umgehen; oder
das ferner ein Ventil umfasst, das an einem Verbinder vor dem wiederverwendbaren Modul positioniert ist, wobei das Ventil den Verbinder, eine Waschleitung und eine Umgehungsleitung verbindet, wobei das Ventil derart für den Verbinder geöffnet und für die Umgehungs- und die Waschleitung geschlossen ist, dass die Strömung durch den Verbinder und durch das wiederverwendbare Modul geleitet wird.

9. Verfahren nach Anspruch 7, das ferner eine Pumpe umfasst, die an der Anreicherungsvorrichtung oder der Waschleitung angebracht ist.

10. Verfahren nach Anspruch 7, das ferner ein Inertgas umfasst, das aus Argon, Luft, gefilterter Luft, Stickstoff und Helium ausgewählt ist, um das Modul auszublasen.

11. Verfahren nach Anspruch 7, wobei die Waschleitungen in eine obere und eine untere Waschleitung unterteilt sind, und
wobei die obere Leitung eine Fluidleitung ist und die untere Leitung eine Gasleitung ist, oder
wobei die obere Leitung eine Gasleitung ist und die untere Leitung eine Fluidleitung ist, oder
wobei sowohl die obere Leitung als auch die untere Leitung Fluidleitungen sind.

## Revendications

1. Cartouche de sorbant, comprenant :
au moins un module réutilisable (131) contenant un ou plusieurs matériaux sorbants rechargeables, et un second module (132) raccordé en série et formant une cartouche de sorbant, l'au moins un module réutilisable ayant un ou plusieurs raccords (133, 134, 135, 136) pouvant être raccordés sélectivement fluidiquement à une partie quelconque d'un chemin d'écoulement de fluide choisi parmi l'une quelconque d'une ligne de lavage (183), d'une conduite de dérivation (180), d'une conduite de fluide ou du second module ;
dans laquelle la ligne de lavage (183) est une ligne pouvant être raccordée fluidiquement à un chargeur (148) pour diriger le fluide entre le chargeur et le module réutilisable ;
dans laquelle la conduite de dérivation (180) est une conduite pouvant être raccordée fluidiquement à un autre module ; et
dans laquelle la conduite de fluide peut être raccordée fluidiquement à n'importe quelle partie d'un circuit de dialyse ; et
dans laquelle le chargeur contient des solutions permettant de recharger le matériau sorbant à l'intérieur du module réutilisable ; ladite cartouche de sorbant comprenant en outre au moins une vanne (174, 175, 176, 177) positionnée avant et/ou après les modules sur les raccords pour diriger sélectivement le flux à travers au moins un module, une ligne de lavage, un chargeur ou une conduite de dérivation ; dans laquelle le module réutilisable est conçu pour être dans un état en ligne en étant raccordé fluidiquement à l'une quelconque de la ligne de fluide ou de la conduite de dérivation, et dans laquelle le module réutilisable est conçu pour être dans un état hors ligne en étant raccordé fluidiquement au chargeur,
dans laquelle « en ligne » fait référence à un état dans lequel le module est raccordé fluidiquement à une machine de dialyse, un chemin d'écoulement de dialyse ou un circuit de dialyse, et dans laquelle « hors ligne » fait référence à un état dans lequel le module est désaccouplé fluidiquement d'une machine de dialyse, d'un chemin d'écoulement ou d'un circuit de dialyse.

2. Cartouche de sorbant selon la revendication 1, dans laquelle : une première conduite de dérivation (180) raccorde une première vanne positionnée sur un connecteur avant le module réutilisable à une deuxième vanne (175) ; une deuxième conduite de dérivation (181) raccorde la deuxième vanne à une troisième vanne (176) positionnée sur un raccord entre le module réutilisable et le second module ; et une troisième conduite de dérivation (182) raccorde la deuxième vanne à une quatrième vanne (177) positionnée sur un raccord après le second module ; ou
dans laquelle une conduite de dérivation (180) raccorde une première vanne (174) positionnée sur un raccord avant le module réutilisable à une deuxième vanne (175) positionnée sur un raccord entre le premier et le second module, et raccorde la deuxième vanne à une troisième vanne (176) positionnée sur un raccord après le second module, la cartouche de sorbant comprenant en outre l'une quelconque d'une vanne à quatre voies, trois voies, deux voies, ou des combinaisons de celles-ci, positionnée avant et/ou après les modules sur les raccords pour sélectivement diriger l'écoulement de fluide à travers les modules, la ligne de lavage, le chargeur et/ou la conduite de dérivation ; ou
dans laquelle une première conduite de dérivation connecte une première vanne (174) positionnée sur un raccord avant le module réutilisable à une deuxième vanne (175) positionnée sur un connecteur entre le module réutilisable et le second module ; et une seconde conduite de dérivation (181) raccorde une troisième vanne (176) positionnée sur le raccord entre le module réutilisable et le second module à une quatrième vanne (177) positionnée sur un raccord après le second module ; ou
dans laquelle une première conduite de dérivation (180) raccorde une première vanne (174) positionnée sur un raccord avant le module réutilisable à une deuxième vanne (175) positionnée sur un raccord entre les premier et second modules ; une seconde conduite de dérivation (181) raccorde une troisième vanne (176) positionnée sur le raccord entre le module réutilisable et le second module à une quatrième vanne (177) positionnée sur un raccord après le second module ; une première ligne de lavage (183) raccorde la première vanne à un premier raccord de chargeur ; une deuxième ligne de lavage (184) raccorde la deuxième vanne à un deuxième raccord de chargeur ; une troisième ligne de lavage (185) raccorde la troisième vanne à un troisième connecteur de chargeur ; et une quatrième ligne de lavage (186) raccorde la quatrième vanne à un quatrième raccord de chargeur ; ou
dans laquelle une première vanne (174) positionnée sur un connecteur avant que le module réutilisable raccorde une première ligne de lavage (183) à un premier connecteur de chargeur ; une deuxième vanne (175) positionnée sur un raccord entre le module réutilisable et le second module raccorde une deuxième ligne de lavage (184) à un deuxième raccord de chargeur ; une troisième vanne (176) positionnée sur le raccord entre les premier et second modules raccorde une troisième ligne de lavage (185) à un troisième raccord de chargeur ; une quatrième vanne (177) positionnée sur un raccord après que le second module raccorde une quatrième ligne de lavage (186) à un quatrième raccord de chargeur ; une cinquième vanne (178) positionnée sur le raccord avant le module réutilisable raccorde une conduite de dérivation à une sixième vanne (179) positionnée sur le raccord entre le module réutilisable et le second module, et se raccorde en outre à une septième vanne positionnée sur le raccord après le second module.

3. Cartouche de sorbant selon la revendication 1 ou 2, dans laquelle les modules réutilisables et le second module font partie d'un circuit de dialyse conforme commandé (380).

4. Cartouche de sorbant selon l'une quelconque des revendications précédentes, dans laquelle les vannes sont actionnées sous la commande d'un contrôleur programmable ou d'un système informatique pour réguler l'écoulement entrant, sortant et entre les modules.

5. Cartouche de sorbant selon l'une quelconque des revendications précédentes, dans laquelle l'écoulement de fluide à travers les vannes est détecté par une cellule photoélectrique ou un autre appareil de détection d'écoulement et/ou de mesure d'écoulement (348).

6. Cartouche de sorbant selon l'une quelconque des revendications précédentes, comprenant en outre une pompe de commande (375) pour faire circuler le fluide dans le chemin d'écoulement de fluide.

7. Procédé de recharge d'un sorbant dans une cartouche de sorbant, comprenant au moins un module réutilisable contenant un ou plusieurs matériaux sorbants rechargeables, et un second module, l'au moins un module réutilisable ayant un ou plusieurs raccords pouvant être raccordés
sélectivement de manière fluidique à n'importe quelle partie d'un chemin d'écoulement de fluide sélectionné parmi l'une quelconque d'une ligne de lavage, une conduite de dérivation, une ligne de fluide ou le second module, dans laquelle le procédé de recharge du sorbant comprend les étapes consistant à :
raccorder au moins un module réutilisable et le second module en série avec un ou plusieurs raccords ; sélectivement :
raccorder de manière fluidique au moins un connecteur à au moins une ligne de lavage, dans laquelle la ligne de lavage est raccordée fluidiquement à un chargeur contenant des solutions destinées à recharger le matériau sorbant à l'intérieur du module réutilisable ;
raccorder fluidiquement au moins un raccord à une conduite de dérivation, la conduite de dérivation détournant l'écoulement depuis le raccord pour dériver au moins un module ;
raccorder une ou plusieurs vannes aux raccords au niveau des jonctions entre les modules, les lignes de lavage et/ou les conduites de dérivation ; et
ouvrir et fermer sélectivement les vannes pour diriger sélectivement l'écoulement à travers les raccords, les modules, les lignes de lavage et/ou les conduites de dérivation, pour mettre le module réutilisable dans un état hors ligne en étant raccordé fluidiquement au chargeur et pour mettre le module réutilisable dans un état en ligne en étant raccordé fluidiquement à l'une quelconque de la ligne de fluide ou de la conduite de dérivation,
dans laquelle « en ligne » fait référence à un état dans lequel le module est raccordé fluidiquement à une machine de dialyse, un chemin d'écoulement de dialyse ou un circuit de dialyse, et dans laquelle « hors ligne » fait référence à un état dans lequel le module est désaccouplé fluidiquement d'une machine de dialyse, d'un chemin d'écoulement ou d'un circuit de dialyse.

8. Procédé selon la revendication 7, comprenant en outre une vanne positionnée sur un raccord avant le module réutilisable, la vanne raccordant le raccord, une ligne de lavage et une conduite de dérivation, dans laquelle la vanne est ouverte à la ligne de lavage, et fermée au raccord et la conduite de dérivation de telle sorte que l'écoulement est dirigé vers un chargeur ; ou
comprenant en outre une vanne positionnée sur un raccord avant un module réutilisable, la vanne raccordant le raccord, une ligne de lavage et une conduite de dérivation, la vanne étant ouverte à la ligne de lavage et au raccord, et fermée à la conduite de dérivation de telle sorte que l'écoulement circule entre le module réutilisable et le chargeur, mais dans laquelle une deuxième vanne positionnée sur un raccord entre le module réutilisable et un second module est fermée de telle sorte que l'écoulement ne peut pas continuer du module réutilisable au second module ; ou
comprenant en outre une vanne positionnée sur un raccord avant le module réutilisable, la vanne raccordant le raccord, une ligne de lavage et une conduite de dérivation, la vanne étant ouverte à la conduite de dérivation et fermée à la ligne de lavage et au raccord, de telle sorte que l'écoulement est dirigé à travers la conduite de dérivation pour dériver le module réutilisable ; ou
comprenant en outre une vanne positionnée sur un raccord avant le module réutilisable, la vanne raccordant le connecteur, une ligne de lavage et une conduite de dérivation, la vanne étant ouverte au raccord et fermée aux conduites de dérivation et lignes de lavage, de telle sorte que l'écoulement est dirigé à travers le raccord et à travers le module réutilisable.

9. Procédé selon la revendication 7, comprenant en outre une pompe fixée au chargeur ou à la ligne de lavage.

10. Procédé selon la revendication 7, comprenant en outre un gaz inerte choisi parmi l'un quelconque de l'argon, de l'air, de l'air filtré, de l'azote et de l'hélium pour souffler le module.

11. Procédé selon la revendication 7, dans lequel les lignes de lavage sont subdivisées en une ligne de lavage supérieure et une ligne de lavage inférieure, et
dans lequel la ligne supérieure est une ligne de fluide et la ligne inférieure est une ligne de gaz, ou
dans lequel la ligne supérieure est une ligne de gaz et la ligne inférieure est une ligne de fluide, ou
dans lequel la ligne supérieure et la ligne inférieure sont toutes deux des lignes fluide.
